# EUROPEAN PATENT APPLICATION

(11) **EP 0 810 227 A1**
(43) Date of publication of application: **03.12.1997**
(21) Application number: 96901987.6
(22) Date of filing: 13.02.1996
(51) Int. Cl.: C07D 413/06, A01N 43/80

(54) **ISOXAZOLE DERIVATIVES**

(30) Priority: 15.02.1995 JP 26427/95; 30.10.1995 JP 281893/95
(71) Applicant: IDEMITSU KOSAN COMPANY LIMITED, Tokyo 100 (JP)
(72) Inventor: TAKASHIMA, Yoriyuki, Sodegaura-shi Chiba-ken 299-02 (JP); KOIKE, Kazuyoshi, Sodegaura-shi Chiba-ken 299-02 (JP); YOSHIKAWA, Misako, Sodegaura-shi Chiba-ken 299-02 (JP); NAKAMURA, Kazufumi, Sodegaura-shi Chiba-ken 299-02 (JP)
(74) Representative: Türk, Gille, Hrabal, Leifert
(86) International application number: JP9600299
(87) International publication number: WO9625413

(57) **Abstract**

The present invention relates to a new isoxazole derivative represented by the formula (I).

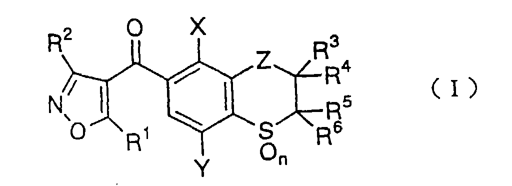

Said isoxazole derivative which causes no phytotoxicity on useful crops such as corn, cotton, wheat and barley but can selectively simultaneously control gramineous weeds and broad-leaved weeds as a single herbicide at a low dosage, and a herbicide containing the above isoxazole derivative as an active ingredient.

## Description

### Technical Field

The present invention relates to novel isoxazole derivatives and herbicides containing them as active ingredients.

### Background Art

During the planting time of corn, etc., a triazine-based herbicide such as atrazine and acid anilide-based herbicides such as alachlor and methlachlor have been conventionally used. However, atrazine shows low efficacy to gramineous weeds, and on the other hand, alachlor and methalachlor show low efficacy to broad-leaved weeds. It is therefore difficult at present to control gramineous weeds and broad-leaved weeds together simultaneously with a single herbicide. Further, even a mixture of these herbicides produces no sufficient herbicidal efficacy, and these herbicides are undesirable in view of an environmental problem due to their high dosage requirement.

On the other hand, during the planting time of cotton, trifluralin, a dinitroaniline-based herbicide, fluometuron, a urea-based herbicide and norflurazon, a pyridazine-based herbicide have been used as an agent for soil treatment. Further, during the planting time of wheat or barley, urea-based herbicides such as isoproturon and chlorotoluron have been used for controlling gramineous weeds in particular. However, these herbicides are also environmentally undesirable due to their high dosage requirement.

It is known that specific isoxazole derivatives have herbicidal activity (JP-A-5-255284, WO94/18179).

Compound 2 disclosed in WO94/18179, a typical example of the isoxazole derivatives disclosed in the above publications, has the following structural formula.

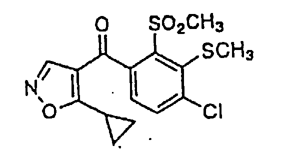

Concerning a compound having a thiochroman ring and having herbicidal activity, compounds in which a thiochroman ring and a pyrazole ring bond to each other are known as pyrazole derivatives, and International Laid-open Patent Publication WO93/18031 of PCT application filed by Applicant discloses, for example, a compound (Compound No. 68) of the following formula.

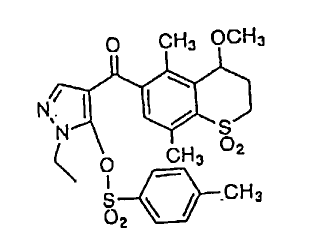

Further, the following recent laid-open publications disclose herbicide compositions having structures closer to the isoxazole derivatives of the present invention. That is, EP95/0636622A1 (JP-A-7-149742) discloses isoxazole derivatives having a structure in which an isoxazole group and a benzo-condensed type bicyclic group bond to each other and having herbicidal activity, and it describes as a specific example of the bicyclic group, a thiochroman ring which is part of the skeleton of the compounds of the present invention. However, the above publication describes no specific "Example" concerning the compound having a thiochroman ring. It discloses compounds having a benzo-condensed type bicyclic group structurally similar to a thiochroman ring, for example, a compound (Compound No. 25) of the following formula.

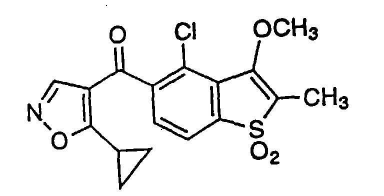

However, the compounds disclosed in the above publication show practically insufficient efficacy as herbicides.

It is therefore an object of the present invention to provide a novel isoxazole derivative which causes no phytotoxicity on useful crops such as corn, cotton, wheat and barley but can selectively simultaneously control gramineous weeds and broad-leaved weeds as a single herbicide at a low dosage, and a herbicide containing the above isoxazole derivative as an active ingredient.

### Disclosure of the Invention

The first essence of the present invention resides in an isoxazole derivative of the general formula (I), 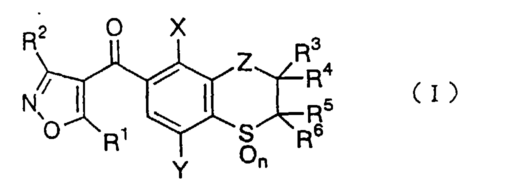 wherein:
R¹ is a C₁-C₆ alkyl group or C₃-C₆ cycloalkyl group,
R² is a hydrogen atom or a C₁∼C₄ alkoxycarbonyl group,
each of R³ ∼ R⁶ is independently a hydrogen atom or a C₁∼C₄ alkyl group,
n is an integer of 0, 1 or 2,
X is a hydrogen atom or a C₁∼C₄ alkyl group,
Y is a hydrogen atom, a C₁∼C₄ alkyl group or a halogen atom, and
Z is a group of 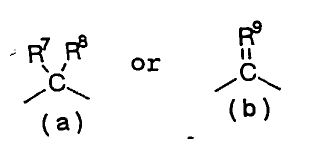
in which each of R⁷ and R⁸ is independently a hydrogen atom, a C₁∼C₄ alkyl group or a C₁∼C₄ alkoxy group, provided that when R⁷ and/or R⁸ are/is C₁∼C₄ alkyl group(s) or C₁∼C₄ alkoxy group(s), 1 to 9 halogen atoms may be substituted on carbon atoms of the substituents, that when the number of carbon atoms of each substituent is C₂∼C₄, each substituent may have an unsaturated bond, and that when both R⁷ and R⁸ are C₁∼C₄ alkyl groups or C₁∼C₄ alkoxy groups, a combination of these substituents may be a 3- to 7-membered ring in which carbon atoms of the substituents bond to each other, and R⁹ is an oxygen atom, a sulfur atom or a C₁∼C₄ alkoxyimino group, provided that when R⁹ is a C₁∼C₄ alkoxyimino group, 1 to 9 halogen atoms may be substituted on carbon atoms of the substituent and that when the number of carbon atoms of the substituent is C₂∼C₄, the substituent may have an unsaturated bond (the above isoxazole derivative to be sometimes referred to as "isoxazole derivative (I) of the present invention" hereinafter).

The second essence of the present invention resides in an isoxazole derivative of the general formula (Ia), 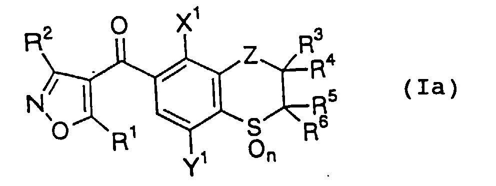 wherein:
R¹ is a C₁-C₆ alkyl group or C₃-C₆ cycloalkyl group,
R² is a hydrogen atom or a C₁∼C₄ alkoxycarbonyl group,
each of R³ ∼ R⁶ is independently a hydrogen atom or a C₁∼C₄ alkyl group,
n is an integer of 0, 1 or 2,
X¹ is a C₁∼C₄ alkyl group,
Y¹ is a C₁∼C₄ alkyl group or a halogen atom, and
Z is a group of 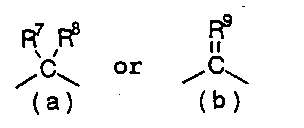
in which each of R⁷ and R⁸ is independently a hydrogen atom, a C₁∼C₄ alkyl group or a C₁∼C₄ alkoxy group, provided that when R⁷ and/or R⁸ are/is C₁∼C₄ alkyl group(s) or C₁∼C₄ alkoxy group(s), one to 9 halogen atoms may be substituted on carbon atoms of the substituents, that when the number of carbon atoms of each substituent is C₂∼C₄, each substituent may have an unsaturated bond, and that when both R⁷ and R⁸ are C₁∼C₄ alkyl groups or C₁∼C₄ alkoxy groups, a combination of these substituents may be a 3- to 7-membered ring in which carbon atoms of the substituents bond to each other, and R⁹ is an oxygen atom, a sulfur atom or a C₁∼C₄ alkoxyimino group, provided that when R⁹ is a C₁∼C₄ alkoxyimino group, one to 9 halogen atoms may be substituted on carbon atoms of the substituent and that when the number of carbon atoms of the substituent is C₂∼C₄, the substituent may have an unsaturated bond.

The third essence of the present invention resides in an isoxazole derivative of the general formula (Ib), 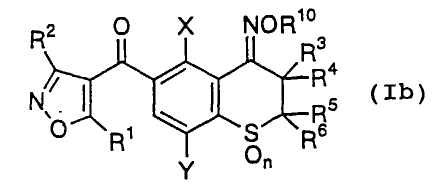 wherein:
R¹ is a C₁-C₆ alkyl group or a C₃-C₆ cycloalkyl group,
R² is a hydrogen atom or a C₁∼C₄ alkoxycarbonyl group,
each of R³ ∼ R⁶ is independently a hydrogen atom or a C₁∼C₄ alkyl group,
n is an integer of 0, 1 or 2,
X is a hydrogen atom or a C₁∼C₄ alkyl group,
Y is a hydrogen atom, a C₁∼C₄ alkyl group or a halogen atom, and
R¹⁰ is a C₁∼C₄ alkyl group provided that one to 9 halogen atoms may be substituted on carbon atoms of the substituent and that when the number of carbon atoms of the substituent is C₂∼C₄, the substituent may have an unsaturated bond.

The fourth essence of the present invention resides in a herbicide containing the above isoxazole derivative as an active ingredient (to be sometimes referred to as "herbicide of the present invention" hereinafter).

### Best Mode for Carrying Out the invention

The isoxazole derivative of the present invention has the following general formula (I).

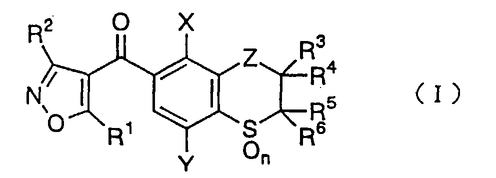

In the above general formula (I), R¹ is a C₁-C₆ alkyl group or a C₃-C₆ cycloalkyl group. The C₁-C₆ alkyl group includes methyl, ethyl, propyl, butyl, pentyl and hexyl. The propyl, butyl, pentyl and hexyl may be linear or branched. A C₁∼C₄ alkyl group is preferred, methyl, ethyl or i-propyl is more preferred, and methyl is particularly preferred. The C₃-C₆ cycloalkyl group includes cyclopropyl, cyclobutyl, cyclopentyl and cyclohexyl, and cyclopropyl is preferred.

R² is a hydrogen atom or a C₁∼C₄ alkoxycarbonyl group. The C₁∼C₄ alkoxycarbonyl group includes methoxycarbonyl, ethoxycarbonyl, propoxycarbonyl and butoxycarbonyl. The alkyl chain each of the propoxycarbonyl and the butoxycarbonyl may be linear or branched. R² is preferably a hydrogen atom.

X is a hydrogen atom or a C₁∼C₄ alkyl group. The C₁∼C₄ alkyl group includes those as described in the above R¹. X is preferably a hydrogen atom.

Y is a hydrogen atom, a C₁∼C₄ alkyl group or a halogen atom. The C₁∼C₄ alkyl group includes those described concerning the above X. The halogen atom includes fluorine, chlorine, bromine and iodine. Y is preferably hydrogen atom, methyl, fluorine or chlorine, particularly preferably hydrogen atom, methyl or chlorine.

Each of R³, R⁴, R⁵ and R⁶ is independently a hydrogen atom or a C₁∼C₄ alkyl group. The C₁∼C₄ alkyl group includes those described concerning the above X. Preferred is a hydrogen atom or methyl.

n is the number of oxygen atom bonding to the sulfur atom, and it is an integer of 0, 1 or 2. When n = 0, a sulfide is represented. When n = 1, a sulfoxide is represented. When n = 2, a sulfone is represented. n is preferably 2 (sulfone).

Z is a group of

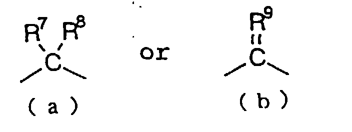

In the above formula (a), each of R⁷ and R⁸ is independently a hydrogen atom, a C₁∼C₄ alkyl group or a C₁∼C₄ alkoxy group. The C₁∼C₄ alkyl group includes those described concerning the above X. When R⁷ and/or R⁸ are/is alkyl, one ∼ 9 hydrogen atoms on the carbon chain may be arbitrarily replaced by halogen atoms, and specific examples of the substituted alkyl include -CH₂F, -CHF₂, -CF₃, CH₂Cl, -CCCl₃, -CH₂Br, -CH₂I, -CH₂CH₂F, -CH₂CF₃, -CF₂CH₃, -CF₂CHF₂, -C₂F₅, -CH₂CH₂Cl, -CH₂CCl₃, -CCl₂CH₃, -C₂Cl₅, -CF(CH₃)₂, -CH(CF₃)CH₃, -CH(CF₃)₂, -CCl(CH₃)₂, -CH(CCl₃)₂, -CH₂CH₂CH₂F, -CH₂CH₂CF₃, -C₃F₇, -CH₂CH₂CH₂Cl, -CHClCH₂CH₃, -CH₂CH₂CH₂CH₂F, -CH₂CH₂CH₂CH₂Cl, -CH(CH₃)CH₂CH₂Cl, -CH(CF₃)C₂H₅, -CF(CH₃)C₂H₅ and -C₄F₉. Preferred are -CHF₂, -CF₃, -CCl₃, -CH₂CH₂F, -CH₂CF₃, -C₂F₅ and -CF(CH₃)₂. Further, when R⁷ and/or R⁸ is a C₂∼C₄ alkyl group, adjacent carbon atoms of the substituent may have a double bond or a triple bond. Specific examples of the C₂∼C₄ alkyl group having a double or triple bond include -CH=CH₂, -CH≡CH, -C(CH₃)=CH₂, -CH=C(CH₃)₂, -CH₂CH=CH₂, -CH(CH₃)CH=CH₂ and -CH₂C≡CH. Preferred are -CH=CH₂, -CH≡CH, -C(CH₃)=CH₂. Further, when each of R⁷ and R⁸ is independently a C₁∼C₄ alkyl group at the same time, an arbitrary carbon atom of R⁷ and an arbitrary carbon atom of R⁸ may bond to each other to form a 3-membered to 7-membered ring. Specific examples of this ring include groups of 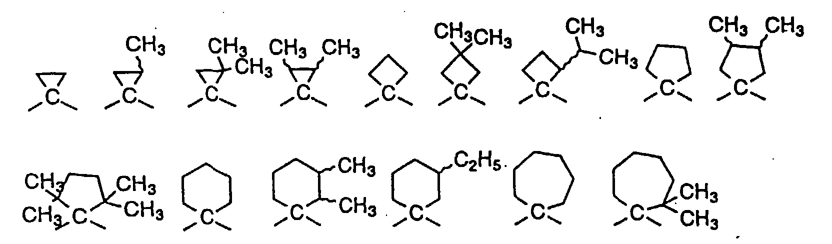 and preferred are groups of

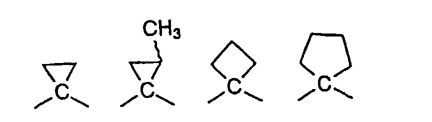

The C₁∼C₄ alkoxy group includes methoxy, ethoxy, propoxy and butoxy. The alkyl chain each of the propoxy and the butoxy may be linear or branched. When R⁷ and/or R⁸ are/is a C₁∼C₄ alkoxy group, one to 7 hydrogen atoms on the carbon chain thereof may be arbitrarily replaced by halogen atom(s). Specific examples of the substituted alkoxy group include -OCF₃, -OCCl₃, -OCH₂CH₂F, -OCH₂CH₂Cl, -OCH₂CH₂Br, -OCH₂CH₂I, -OCH₂CF₃, -OCH₂CCl₃, -OCF(CH₃)₂, -OCH(CF₃)CH₃, -OCH(CF₃)₂, OCH(CCl₃)₂, -OCH₂CH₂CH₂F, -OCH₂CH₂CF₃, -OCH₂CF₂CF₃, -OCH₂CH₂CH₂Cl, -OCH₂CH₂CH₂CH₂F, -OCH₂CH₂CH₂CH₂Cl, -OCH(CH₃)CH₂CH₂Cl and -OCH(CF₃)C₂H₅. Preferred are -OCH₂CH₂F, -OCH₂CH₂Cl, -OCH₂CF₃, -OCF(CH₃)₂, -OCH(CF₃)CH₃ and -OCH(CF₃)₂. -OCH₂CH₂F is more preferred.

Further, when R⁷ and/or R⁸ are/is C₂∼C₄ alkoxy, adjacent carbon atoms of the substituent(s) may have a double bond or triple bond. Specific examples of the alkoxy include -OCH=CH₂, -OC(CH₃)=CH₂, -OCH=C(CH₃)₂, -OCH₂CH=CH₂, -OCH₂C(CH₃)=CH₂, -OCH(CH₃)CH=CH₂ and -OCH₂C≡CH. Preferred are -OCH₂CH=CH₂ and -OCH₂C≡CH.

Further, when each of R⁷ and R⁸ is independently a C₁∼C₄ alkoxy group at the same time, an arbitrary carbon atom of R⁷ and an arbitrary carbon atom of R⁸ may bond to each other to form a 5-membered to 7-membered ring. Specific examples of this ring include groups of 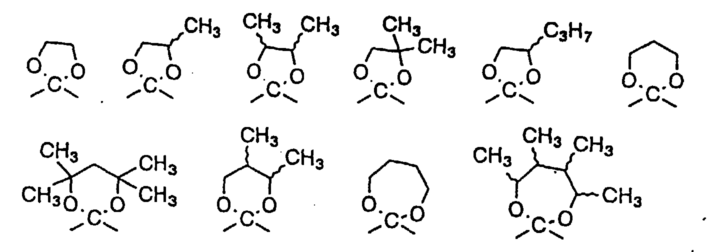 and preferred are groups of

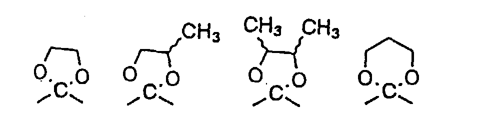

When Z is a group of (a), preferred are a group in which R⁸ is a hydrogen atom and R⁷ is any one of methyl, ethyl, isopropyl, -CF₃, -C₂F₅, -CF(CH₃)₂, -CH=CH₂, -C(CH₃)=CH₂, methoxy, ethoxy, isopropoxy, -OCH₂CH₂F, -OCH₂CH=CH₂ and -OCH₂C≡CH, a group in which R⁷ and R⁸ are the same substituents, i.e., R⁷ and R⁸ are concurrently methyl, -CF₃ or methoxy, and a group which is composed of identical R⁷ and R⁸ bonded to the carbon atom on the 4-position of the thiochroman ring and has a cyclopropane ring, cyclobutane ring, cyclopentane ring or 2,5-dioxolan structure.

In the group (b) for Z, R⁹ is an oxygen atom, a sulfur atom or a C₁∼C₄ alkoxyimino group, and the C₁∼C₄ alkoxyimino group includes methoxyimino, ethoxyimino, propoxyimino and butoxyimino. The alkyl group each of the propoxyimino and the butoxyimino may be linear or branched. When R⁹ is a C₁∼C₄ alkoxyimino group, one to 9 hydrogen atoms on the carbon chain thereof may be arbitrarily replaced by halogen atom(s). Specific examples of the substituted alkoxyimino include =NOCH₂CH₂F, =NOCH₂CH₂Cl, =NOCH₂CH₂Br, =NOCH₂CH₂I, =NOCH₂CF₃, =NOCH₂CCl₃, =NOCF(CH₃)₂, =NOCH(CF₃)CH₃, =NOCH(CF₃)₂, =NOCH(CCl₃)₂, =NOCH₂CH₂CH₂F, =NOCH₂CH₂CF₃, =NOCH₂CF₂CF₃, =NOCH₂CH₂CH₂Cl, -NOCH₂CH₂CH₂CH₂F, =NOCH₂CH₂CH₂CH₂Cl, =NOCH(CH₃)CH₂CH₂Cl and =NOCH(CF₃)C₂H₅. Preferred are =NOCH₂CH₂F, =NOCH₂CH₂Cl, =NOCH₂CF₃, =NOCF(CH₃)₂ and =NOCH(CF₃)CH₃, and =NOCH₂CH₂F is more preferred.

When R⁹ is a C₂∼C₄ alkoxyimino group, adjacent carbon atoms of the substituent may have a double bond or triple bond. Specific examples of the alkoxyimino group include =NOCH=CH₂, =NOC(CH₃)=CH₂, =NOCH=C(CH₃)₂, =NOCH₂CH=CH₂, =NOCH₂C(CH₃)=CH₂, =NOCH(CH₃)CH=CH₂ and =NOCH₂C≡CH. Preferred are =NOCH₂CH=CH₂ and =NOCH₂C≡CH.

When Z is a group (b), preferred is a group in which R⁹ is an oxygen atom, methoxyimino, ethoxyimino, =NOCH₂CH₂F, =NOCH₂CH=CH₂ or =NOCH₂C≡CH.

The isoxazole derivative which is the second essence of the present invention is the compound represented by the formula (Ia).

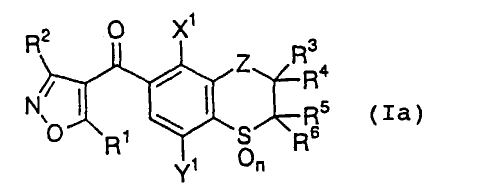

In the formula (Ia), R¹, R², R³, R⁴, R⁵, R⁶, n and Z are as described in the general formula (I).

In the formula (Ia), X¹ is C₁ ∼ C₄ alkyl group, which is the same as descried concerning the group X in the above general formula (I).

In the formula (Ia), Y¹ is C₁ ∼ C₄ alkyl group or halogen atom, both of which are the same as described concerning the group Y in the above general formula (I).

The isoxazole derivative which is the third essence of the present invention is the compound represented by the formula (Ib).

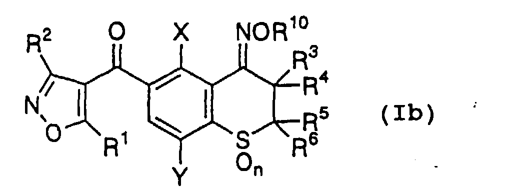

In the formula (Ib), R¹, R², R³, R⁴, R⁵, R⁶, n, X, Y are as described in the general formula (I).

In the formula (Ib), the group corresponding to Z in the formula (I) is 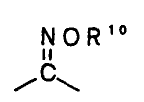 wherein R¹⁰ is C₁ ∼C₄ alkyl group, which is the same as that described in X of the above general formula (I). In the group R¹⁰, carbon atom(s) in the substituent may be substituted by one to 9 halogen atom(s), and unsaturated bond may be formed when the carbon number is C₂ ∼ C₄. The specific examples of R¹⁰ are the same as those described in R⁷ or R⁸ of the above general formula (I).

The isoxazole derivative of the general formula (I) 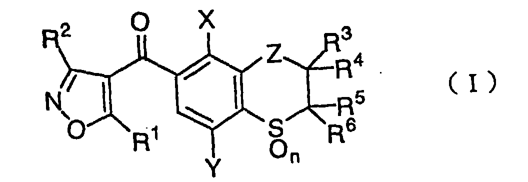 has an asymmetric carbon atom with regard to each of the substituents R³,R⁴, R⁵ and R⁶ and can accordingly include various isomers. The isoxazole derivative of the present invention includes all of these isomers and mixtures of these.

The isoxazole derivative of the general formula (I) in which Z is a group (a), i.e., the isoxazole derivative of the general formula (Ic) 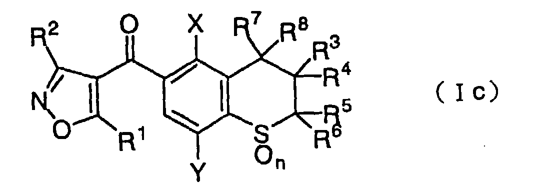 has an asymmetric carbon atom with regard to each of the substituents R⁷ and R⁸ and can accordingly include various isomers. The isoxazole derivative of the present invention includes all of these isomers and mixtures of these.

The isoxazole derivative of the general formula (I) in which Z is a group (b) and R⁹ is a C₁∼C₄ alkoxyimino group, i.e., the isoxazole derivative of the general formula (Ib) 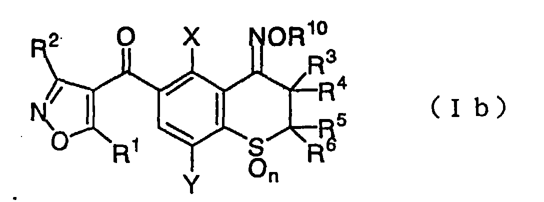 includes geometric isomers of the following general formulae (1b1) and (1b2) with regard to the alkoxyimino group. The isoxazole derivative of the present invention includes one of these isomers or a mixture of these.

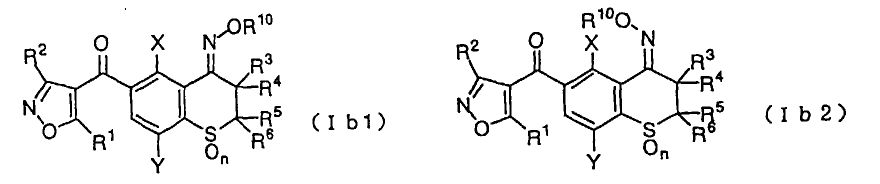

The novel isoxazole derivative of the present invention can be produced according to a known method such as those disclosed in JP-A-3-118374 and JP-A-6-271554. The following reaction scheme is one example.

The process for the production of the compound of the general formula (I) in which R² is a hydrogen atom, i.e., a compound of the general formula (Id), will be explained in detail bellow. 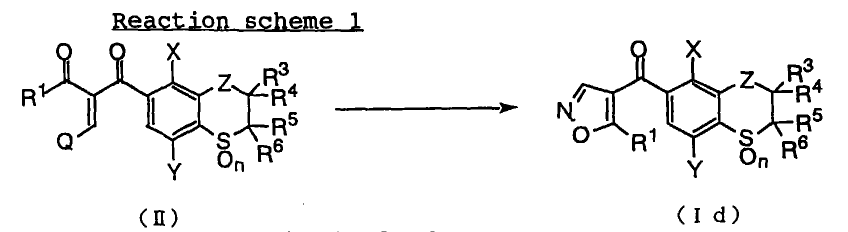 wherein R¹, R³, R⁴, R⁵, R⁶, X, Y, Z and n are as defined in the general formula (I), and Q is a C₁∼C₄ alkoxy group or a C₁∼C₄ dialkylamino group.

The above reaction scheme 1 shows the production of an isoxazole derivative of the general formula (Id), provided by the present invention, by a method in which 2-dialkylaminomethylidene derivative of the general formula (II) (in which Q is dialkylamino) or 2-alkoxymethylidene derivative of the general formula (II) (in which Q is alkoxy) is reacted with a hydroxyamine salt in a solvent such as ethanol or acetonitrile optionally in the presence of a base such as triethylamine or sodium acetate at a temperature in the range of from room temperature to the reflux temperature of the solvent. The isoxazole derivative of the above general formula (Id) is limited to the isoxazole derivative of the general formula (I) in which R² is hydrogen.

After the completion of the reaction, according to a conventional method, the isoxazole derivative of the present invention, synthesized by the above method, is isolated by distilling off the solvent, dissolving the residue in a solvent such as methylene chloride, washing the resultant solution with water, drying the organic layer over a dehydrating agent such as anhydrous sodium sulfate and distilling off the solvent under reduced pressure. Further, the so-obtained residue may be purified by means such as flash column chromatography (Wakogel C300 (trade name):hexane/ethyl acetate = 2:1).

2-Dialkylaminomethylidene derivative or 2-alkylmethylidene derivative of the general formula (II) which is the starting material in Reaction scheme 1 can be produced according to the Reaction scheme 2. 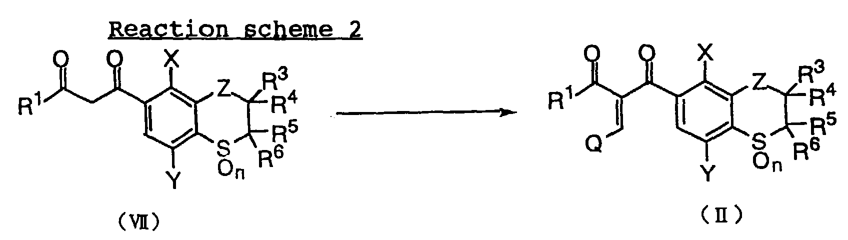 wherein R¹, R³, R⁴, R⁵, R⁶, X, Y, Z and n are as defined in the general formula (I), Hal is a halogen atom and Q is a C₁∼C₄ alkoxy group or a C₁∼C₄ dialkylamino group.

The above Reaction scheme 2 shows the production of 2-alkoxymethylidene derivative (II) (in which Q is alkoxy) by a method in which a 1,3-diketone derivative of the general formula (VII) is reacted with an orthoester such as triethyl orthoformate in an acid catalyst such as acetic anhydride at the reflux temperature of a reaction mixture or the production of 2-dimethylaminomethylidene (II) (in which Q is dialkylamino) by a method in which 1,3-diketone derivative of the general formula (VII) is reacted with an amideacetal such as N,N-dimethylformamidedimethylacetal in an inert solvent such as dioxane at a temperature in the range of from room temperature to the reflux temperature of the solvent. The so-obtained 2-alkoxymethylidene derivative (II) or 2-dimethylaminomethylidene derivative (II) can be used for the synthesis of the isoxazole derivative without their purification.

The method of producing the isoxazole derivative of the general formula (I) in which R² is C₁∼C₄ alkoxycarbonyl, i.e., an isoxazole derivative of the general formula (Ie), will be explained below. 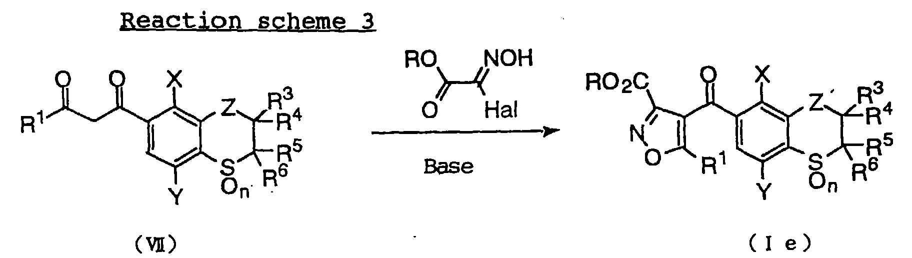 wherein R¹, R³, R⁴, R⁵, R⁶, X, Y, Z and n are as defined in the general formula (I), R is a C₁∼C₄ alkyl group and Hal is a halogen atom.

Reaction scheme 3 shows the production of the isoxazole derivative of the general formula (Ie), provided by the present invention, by a method in which 1,3-diketone derivative of the general formula (VII) is reacted with a compound represented by ROOCC(Hal)=NOH in an inert solvent such as dichloromethane or acetonitrile in the presence of a base such as magnesium ethoxide at a temperature in the range of from room temperature to the reflux temperature of a reaction mixture. The isoxazole derivative of the above general formula (Ie) is limited to the isoxazole derivative of the general formula (I) in which R² is a C₁∼C₄ alkoxycarbonyl group.

The 1,3-diketone derivative of the general formula (VII) which is the starting material in Reaction scheme 3 can be produced according to Reaction scheme 4 or 5. 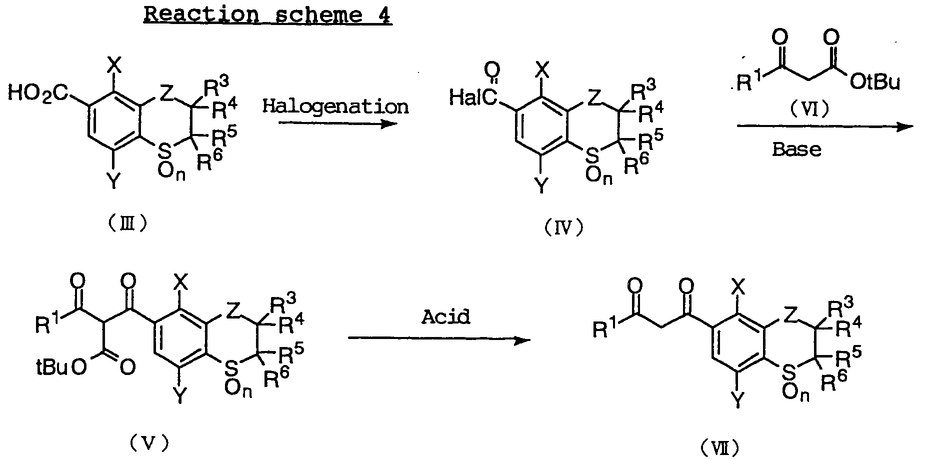 wherein R¹, R³, R⁴, R⁵, R⁶, X, Y, Z and n are as defined in the general formula (I) and Hal is a halogen atom.

Reaction scheme 4 shows the production of the 1,3-diketone derivative of the general formula (VII) by a method including a first step of reacting a carboxylic acid of the general formula (III) with a halogenating agent such as thionyl chloride, oxalyl chloride or phosphorus oxychloride to form carboxylic acid halide of the general formula (IV), a second step of reacting the compound of the general formula (IV) with tert-butylacyl acetate of the general formula (VI) in an inert solvent such as tetrahydrofuran or diethyl ether in the same manner as in the presence of a base such as sodium methoxide or magnesium ethoxide at a temperature in the range of from room temperature to the reflux temperature of the solvent, to form a tert-butyldiketo acetate of the general formula (V), and a third step of decarboxylating the compound of the general formula (V) in the presence of an acid catalyst such as 4-toluenesulfonic acid. 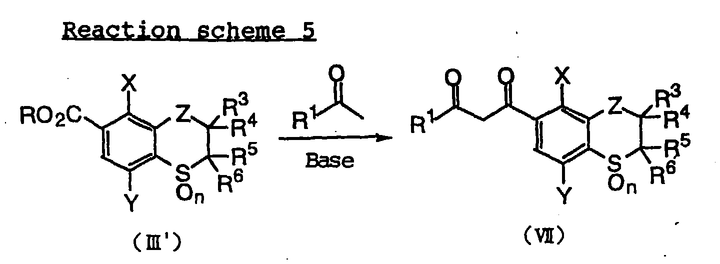 wherein R¹, R³, R⁴, R⁵, R⁶, X, Y, Z and n are as defined in the general formula (I) and R is a C₁∼C₄ alkyl group.

Reaction scheme 5 shows the production of 1,3-diketone derivative of the general formula (VII) by a method in which carboxylic acid ester derivative of the general formula (III') is reacted with R¹COCH₃ in an ether solvent such as tetrahydrofuran or diethyl ether in the presence of a base such as sodium hydroxide or sodium tert-butoxide at a temperature in the range of from 0°C to the reflux temperature of a reaction mixture.

The carboxylic acid ester derivative of the general formula (III') can he produced, for example, by reacting a corresponding carboxylic acid of the general formula (III) with an alcohol represented by ROH (R is C₁ ∼ C₄ alkyl) in the presence of a dehydrating agent such as sulfuric acid at a temperature in the range of from room temperature to the reflux temperature of a reaction mixture according to the following Reaction scheme 6. 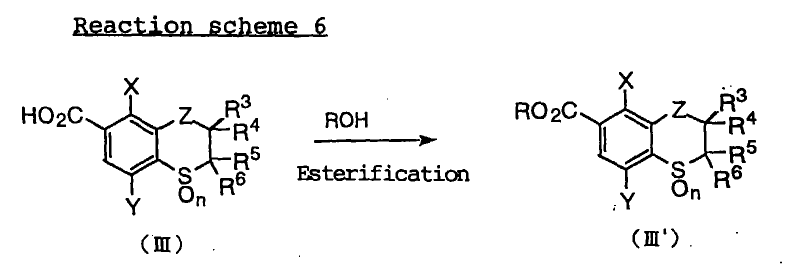 wherein R³, R⁴, R⁵, R⁶, X, Y, Z and n are as defined in the general formula (I) and R is a C₁∼C₄ alkyl group.

The carboxylic acid derivative of the general formula (III) and its carboxylic acid ester derivative of the general formula (III') can be produced by a variety of methods (such as those disclosed, e.g., in WO93/18031, WO94/1431 and WO95/4054).

For example, the above derivatives are specifically produced as follows.

### a) Synthesis of carboxylic acid of the general formula (III) in which Z is a group (a) in which R⁷ is a C₁∼C₄ alkoxy group and R⁸ is a hydrogen atom

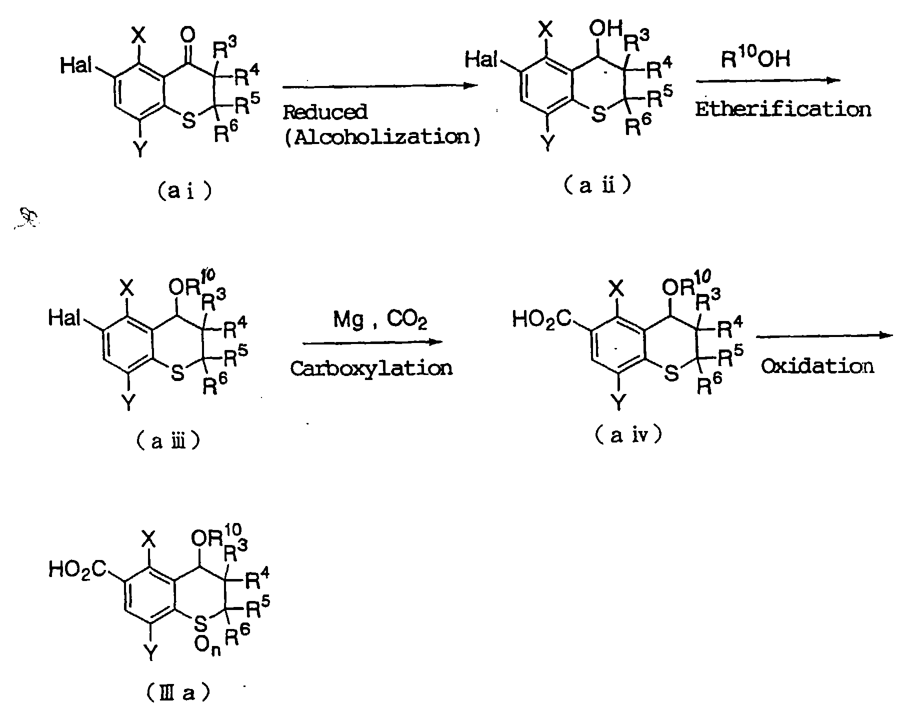

wherein R³, R⁴, R⁵, R⁶, R¹⁰, X, Y and n are as defined in the general formula (I) or the general formula (Ib) and Hal is a halogen atom.

The above method of producing carboxylic acid (IIIa) is disclosed in WO93/18031.

### b) Synthesis of carboxylic acid of the general formula (III) in which Z is a group (b) in which R⁹ is a C₁∼C₄ alkoxy group

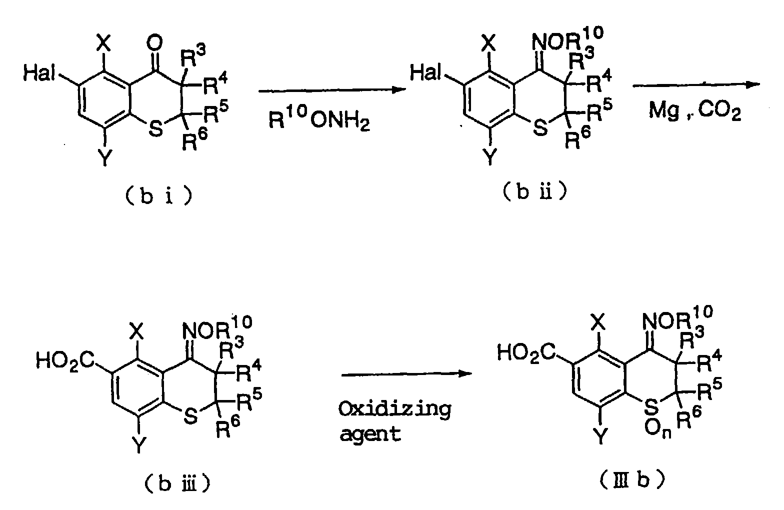

wherein R³, R⁴, R⁵, R⁶, R¹⁰, X, Y and n are as defined in the general formula (I) or the general formula (Ib) and Hal is a halogen atom.

The above method of producing carboxylic acid (IIIb) is disclosed in WO93/1431.

### c) Synthesis of carboxylic acid of the general formula (III) in which Z is a group (a) in which each of R⁷ and R⁸ is a hydrogen atom or a C₁∼C₄ alkyl

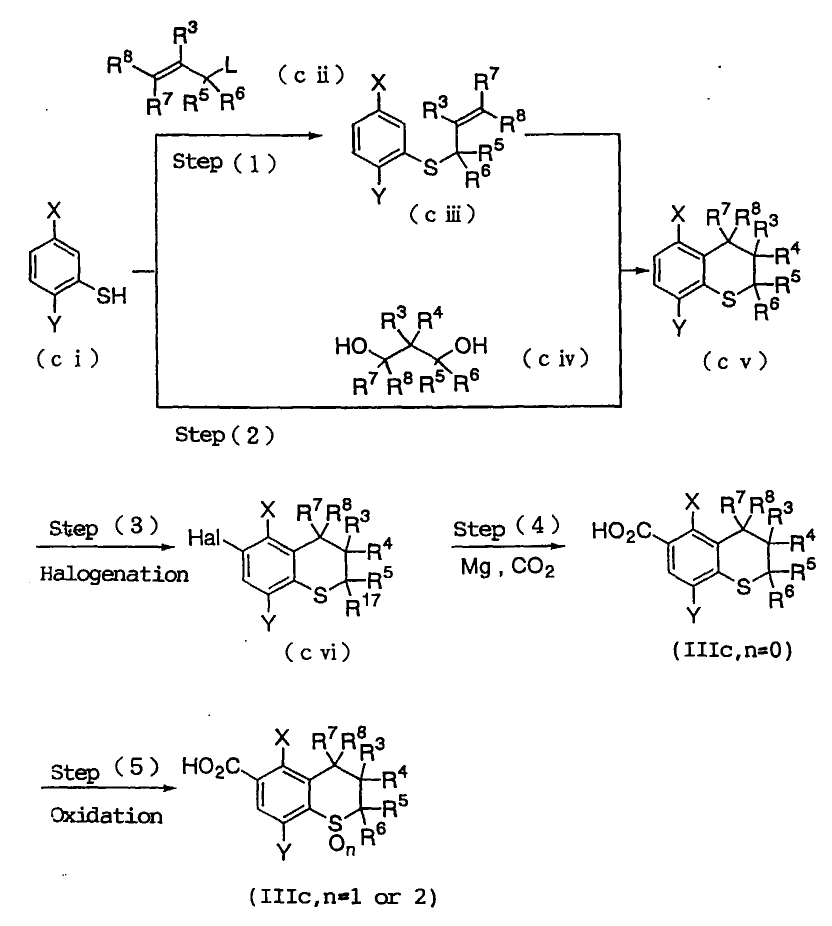

wherein R³, R⁴, R⁵, R⁶, R⁷, R⁸, X, Y and n are as defined in the general formula (I), L is a halogen atom or an OH group and Hal is a halogen atom.

The above method of producing carboxylic acid (IIIc) is disclosed in WO95/04054, and the carboxylic acid (IIIc) can be produced, for example, according to the above method.

Each step of the above method will be explained in detail below.

In addition, thiophenol of the general formula (ci) as a starting material can be prepared by a known method (e.g., "Shin-Jikken Kagaku Koza 14, Syntheses and Reactions of Organic Compounds, III, page 1,704, chap. 8.1, Thiols, f. Synthesis through dithiocarbonate ester", Maruzen, issued Feb. 22, 1986).

Thiochroman derivative of the general formula (cv) can be produced by a method through any one steps (1) and (2).

### Step (1)

### A case where the compound of the general formula (cv) is an allyl halide (L is a halogen atom):

The thiochroman derivative of the general formula (cv) is produced as follows. A thiophenol (ci) and an allyl halide of the general formula (cii) are reacted with each other in an inert solvent such as acetone, diethyl ether or dimethylformamide in the presence of a base such as anhydrous potassium carbonate, sodium hydroxide, potassium hydroxide, anhydrous sodium carbonate or triethylamine at a temperature in the range of from 0°C to the reflux temperature of the solvent, to obtain a sulfide derivative of the general formula (ciii), and further, the sulfide derivative is allowed to react in the presence of polyphosphoric acid, phosphorus pentoxide or an ion-exchange membrane (e.g., Amberlite (trade name)). In this method, R⁴ is limited to a hydrogen atom.

### A case where the compound of the general formula (cii) is an allyl alcohol (L is an OH group):

The thiochroman derivative of the general formula (cv) is produced in one reaction step by condensing a thiophenol (ci) and an allyl alcohol of the general formula (cii) in the presence of an acid catalyst such as formic acid. The formic acid as an acid catalyst may be used as a solvent as well, while other solvent may be used. The solvent is not specially limited. The reaction temperature is properly set in the range of from room temperature to the reflux temperature of the solvent.

### Step (2)

The thiochroman derivative of the general formula (cv) is produced by condensing a thiophenol of the general formula (ci) and an alcohol of the general formula (civ) in the presence of an acid catalyst such as formic acid. The formic acid as an acid catalyst may be used as a solvent as well, while other solvent may be used. The solvent is not specially limited if it is inert to the reaction. The reaction temperature is properly set in the range of from room temperature to the reflux temperature of the solvent.

### Step (3)

A thiochroman derivative of the general formula (cvi) is produced by reacting a compound of the general formula (cv) with a halogenating reagent such as bromine, sulfuryl chloride or chlorine in a solvent such as methylene chloride, chloroform or carbon tetrachloride at a temperature in the range of from 0°C to the reflux temperature of the solvent.

### Step (4)

The carboxylic acid of the general formula (cvii) (in the general formula (IIIc), n=0, sulfide compound) is produced as follows. The thiochroman derivative of the general formula (cvi) is reacted with magnesium in an ether solvent such as tetrahydrofuran or diethyl ether in the copresence of iodine, methyl iodate or ethyl bromide at a temperature in the range of from 0°C to the reflux temperature of the solvent, to obtain a Grignard reagent, and the Grignard reagent is reacted with carbon dioxide (CO₂).

### Step (5)

A carboxylic acid of the general formula (IIIc) is produced by reacting the carboxylic acid of the general formula (cvii) (in the general formula (IIIc), n=0, sulfide compound) with an oxidizing agent such as hydrogen peroxide, peracetic acid, sodium periodate or m-chloroperbenzoate in a solvent such as acetic acid, water, methanol or methylene chloride at a temperature in the range of from -20°C to the reflux temperature of the solvent. For producing a sulfoxide compound (in the formula (IIIc), n=1), preferably, 1 equivalent of the oxidizing agent is used. For producing a sulfone compound (in the formula (IIIc), n=2), at least 2 equivalents of the oxidizing agent is used.

A carboxylic acid of the general formula (IIIc) in which Y is a hydrogen atom, i.e., a carboxylic acid (IIIc_{H}), is produced by reducing 8-chloro-thiochroman derivative (IIIc_{CI}) obtained by the method mentioned below, in a solvent such as ethanol having a water content of 60 % in the presence of a zinc powder. The reaction smoothly proceeds by adding a base such as sodium hydroxide or potassium hydroxide in an amount of at least 1 equivalent. This method is also disclosed in WO95/04054.

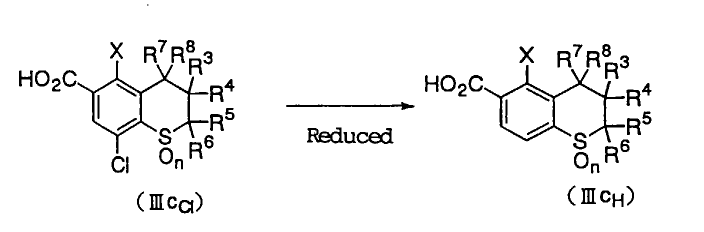

### d) Synthesis of carboxylic acid of the general formula (III) in which Z is a group (b) in which R⁹ is an oxygen atom

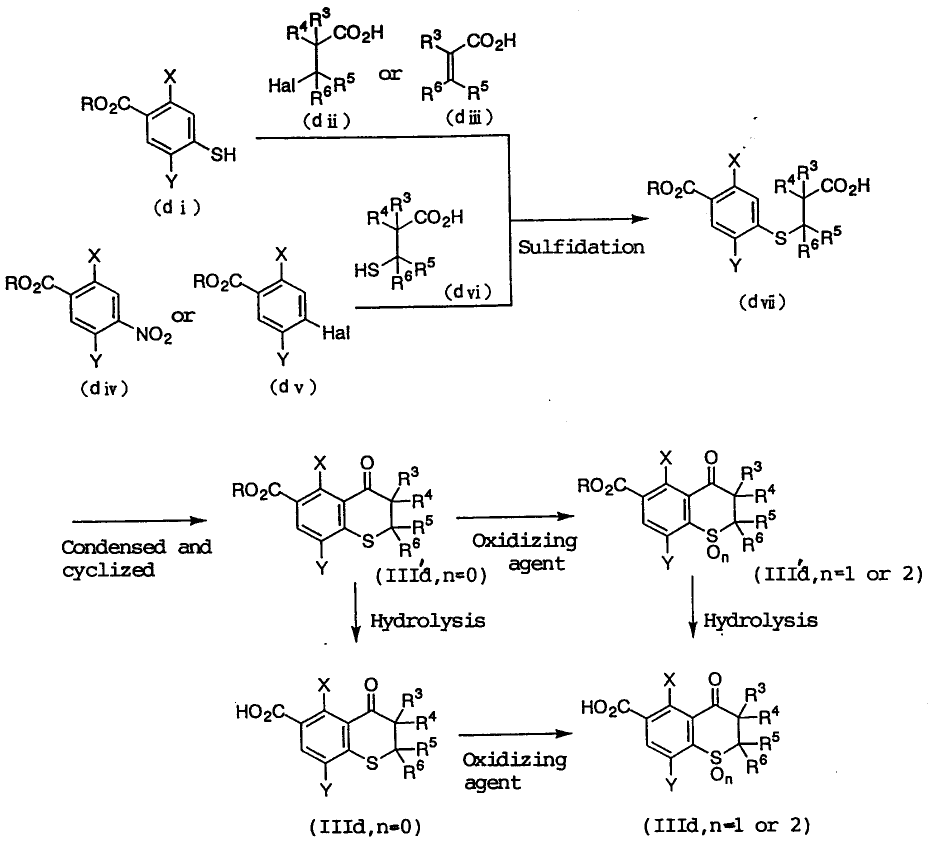

wherein R³, R⁴, R⁵, R⁶, X, Y and n are as defined in the general formula (I), Hal is a halogen atom and R is a C₁∼C₄ alkyl group.

In the above reaction scheme, phenylthiopropionic acid derivative of the general formula (dii) can be synthesized by a method in which p-thiosalicylic acid derivative (di) is bonded to a 3-halopropionic acid compound (dii) or to an acrylic acid compound (diii) or by a method in which a 3-mercaptopropionic acid compound (dvi) is reacted with p-nitrobenzoic acid derivative (div) or with p-halobenzoic acid acid compound (diii) or by a method in which a 3-mercaptopropionic acid compound (dvi) is reacted with p-nitrobenzoic acid derivative (div) or with p-halobenzoic acid derivative (dv).

The above-obtained phenylthiopropionic acid derivative (dvii) is condensed and cyclized in the presence of a dehydrating agent such as polyphosphoric acid, to give a carboxylic acid ester of the general formula (III'd) in which n = 0. This compound is properly oxidized by the same method as that in step (5) in the above reaction scheme (c), to give a carboxylic acid ester of the general formula (III'd) in which n=1 or 2. Further, the each ester is hydrolyzed according to a conventional method to give a carboxylic acid of the general formula (IIId).

A carboxylic acid of the general formula (IIId) in which Y is a hydrogen atom is obtained, for example, by catalytically reducing and hydrogenating the carboxylic acid derivative in which Y is a chlorine atom in the presence of a catalyst under the current of hydrogen gas.

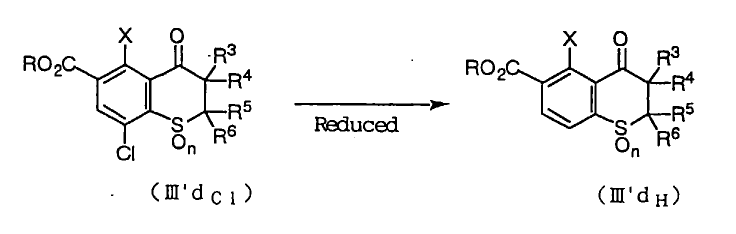

### e) Derivation of compounds having various substituents as Z from carboxylic acid of the general formula (IIId) as raw material

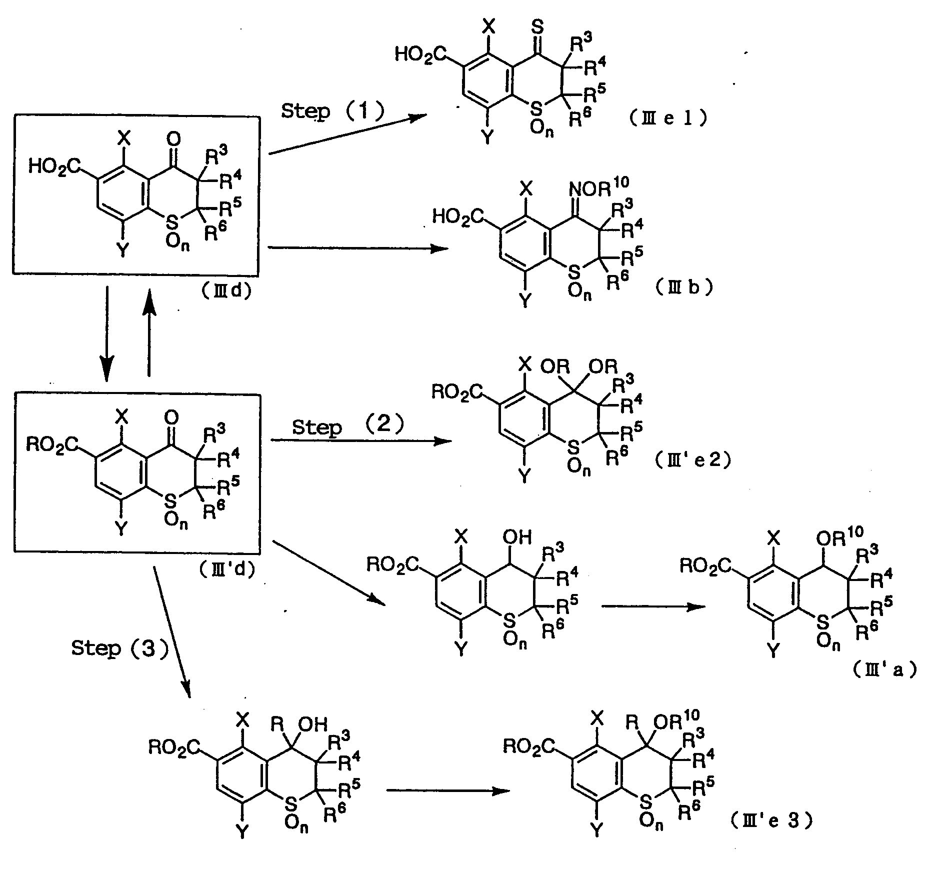

wherein R³, R⁴, R⁵, R⁶, R¹⁰, X, Y and n are as defined in the general formula (I) and the general formula (Ib) and R is a C₁∼C₄ alkyl group.

Various thiochroman carboxylic acids and/or their esters of the above general formulae (IIIe1), (III'e2) and (III'e3) can be derived from the carboxylic acid of the general formula (IIId). Each derivation method is as follows.

### 1. Step (1)

Thiochroman-4-thione derivative of the general formula (IIIe1) is obtained by heating a combination of the carboxylic acid of the general formula (IIId) with a sulfating agent such as disphosphorus pentasulfide or a Lawesson's reagent in an inert solvent.

### 2. Step (2)

Thiochroman-4,4-dialkoxy derivative of the general formula (III'e2) is obtained by heating a combination of the carboxylic acid ester of the general formula (III'd) with a corresponding alcohol in an inert solvent in the presence of a Lewis acid such as p-toluenesulfonic acid or titanium tetrachloride. When the alcohol is selected from alkylene diols such as ethylene glycol, the reaction proceeds relatively smoothly. In the 4,4-dialkoxy compound formed in the case, two substituents (R) in the general formula (III'e) have a cyclized form in which the two alkyl substituents bond to each other.

### 3. Step (3)

Thiochroman-4-alkyl-4-alkoxy compound of the general formula (III'e3) is obtained by reacting the carboxylic acid ester compound of the general formula (III'd) with alkylmagnesium halide (R-Mg-Hal) to obtain a 4-alkyl-4-hydroxy compound as an intermediate, and etherifying this compound, for example, by the method described in the above reaction scheme (a).

### 4. Others

The carboxylic acid ester compound of the general formula (III'a) can be derived from the carboxylic acid ester compound of the general formula (III'd) by the same method as that described in the above reaction scheme (a).

Further, the carboxylic acid of the general formula (IIIb) can be derived from the carboxylic acid of the general formula (IIId) by the same method as that in the above reaction scheme (b).

The herbicide of the present invention contains, as an active ingredient, the isoxazole derivative of the general formula (I) provided by the present invention. The isoxazole derivative of the present invention is mixed with a liquid carrier such as a solvent or a solid carrier such as a mineral fine powder, and the mixture can be prepared into the form of a wettable powder, an emulsifiable concentrate, a dust, granules, or the like. For imparting the preparation with emulsifiability, dispersibility ad spreadability, a surfactant can be added.

When the herbicide of the present invention is used in the form of a wettable powder, generally, a composition is prepared by mixing 10 to 55 % by weight of the isoxazole derivative of the present invention, 40 to 88 % by weight of a solid carrier and 2 to 5 % by weight of a surfactant, and the composition can be used as a wettable powder. Further, when it is used in the form of an emulsifiable concentrate, generally, it can be prepared by mixing 20 to 50 % by weight of the isoxazole derivative of the present invention, 35 to 75 % by weight of a solvent and 5 to 15% by weight of a surfactant.

On the other hand, when herbicide of the present invention is used in the form of a dust, generally, it can be prepared by mixing 1 to 15 % by weight of the isoxazole derivative of the present invention, 80 to 97 % by weight of a solid carrier and a 2 to 5 % by weight of a surfactant. Further, when it is used in the form of granules, it can be prepared by mixing 1 to 15 % by weight of the isoxazole derivative of the present invention, 80 to 97 % by weight of a solid carrier and 2 to 5 % by weight of a surfactant. The above solid carrier can be selected from mineral fine powders, and the mineral fine powders include oxides such as diatomaceous earth and slaked lime, phosphates such as apatite, sulfates such as gypsum, and silicates such as talc, pyroferrite, clay, kaolin, bentonite, acid clay, white carbon, powdered quartz and powdered silica.

The solvent is selected from organic solvents. Specific examples of the solvent include aromatic hydrocarbons such as benzene, toluene and xylene, chlorinated hydrocarbons such as o-chlorotoluene, trichloromethane and trichloroethylene, alcohols such as cyclohexanol, amyl alcohol and ethylene glycol, ketones such as isophorone, cyclohexanone and cyclohexenyl-cyclohexanone, ethers such as butyl cellosolve, diethyl ether and methyl ethyl ether, esters such as isopropyl acetate, benzyl acetate and methyl phthalate, amides such as dimethylformamide, and mixtures of these.

Further, the surfactant can be selected from anionic, nonionic, cationic and amphoteric ones (amino acid and betaine).

The herbicide of the present invention may contain, as active ingredients, other herbicidally active component in combination with the isoxazole derivative of the general formula (I) as required. The "other" herbicidally active component can be selected from phenoxy-, diphenyl ether-, triazine-, urea-, carbamate-, thiocarbamate-, acid anilide-, pyrazole-, phosphoric acid-, sulfonylurea- and oxadiazone-based herbicides as required.

Further, the herbicide of the present invention may be used as a mixture with any one of insecticides, bactericides, plant growth regulators and fertilizers.

### Examples

The present invention will be specifically explained with reference to Referential Preparation Examples of carboxylic acid (III) and starting material (II) and Examples of the isoxazole derivative of the present invention, while the present invention shall not be limited thereto.

### A. Referential Preparation Examples of carboxylic acid (IIIc) Referential Preparation Example 1:

### Synthesis of 4,4,5,8-tetramethylthiochroman-1,1-dioxide-6-carboxylic acid

### (1-1) Synthesis of 4,4,5,8-tetramethylthiochroman

20 ml of formic acid and 0.95 g (0.011 mol) of 3-methyl-2-buten-1-ol were added to 1.79 g (0.01 mol) of 2,5-dimethylthiophenol, and the mixture was refluxed under heat for 7 hours. The reaction mixture was poured into ice water and extracted with ethyl acetate, and then the organic layer was washed with a saturated sodium hydrogencarbonate aqueous solution. The resultant product was dried over anhydrous sodium sulfate, the solvent was distilled off, and the resultant oil was purified by silica gel column chromatography to give 0.95 g (yield 46 %) of the subject end product.
¹H-NMR (ppm, solvent: CDCl₃, internal standard: tetramethylsilane): 1.40 (6H,s), 1.95 ∼ 2.15 (2H,m), 2.20 (3H,s), 2.50 (3H,s), 2.80 ∼ 3.10 (2H,m), 6.80(2H,dd)

### (1-2) Synthesis of 4,4,5,8-tetramethyl-6-bromothiochroman

100 ml of methylene chloride was added to 13.2 g (0.064 mol) of 4,4,5,8-tetramethylthiochroman, 10.2 g (0.064 mol) of bromine was dropwise added at room temperature, and then the mixture was allowed to react for 2 hours. After the completion of the reaction, 70 ml of a 2 % sodium hydrogensulfite aqueous solution was added to the reaction mixture to remove excessive bromine, and the organic layer was washed with a saturated sodium hydrogencarbonate aqueous solution and then with a saturated sodium chloride aqueous solution, and dried over anhydrous sodium sulfate. The solvent was distilled off under reduced pressure, and the resultant oil was purified by silica gel column chromatography to give 1.59 g (yield 87 %) of the subject end product.
¹H-NMR (ppm, solvent: CDCl₃, internal standard: tetramethylsilane): 1.45 (6H,s), 1.90 ∼ 2.10 (2H,m), 2.20 (3H,s), 2.50 (3H,s), 2.80 ∼ 3.00 (2H,m), 7.20(1H,s)

### (1-3) Synthesis of 4,4,5,8-tetramethylthiochroman-6-carboxylic acid

2.9 g (0.12 mol) of magnesium was dispersed in 100 ml of THF (tetrahydrofuran), and 7.4 g (0.068 mol) of ethyl bromide was dropwise added. The mixture was refluxed for 10 minutes, and a solution of 9.7 g (0.034 mol) of 4,4,5,8-tetramethyl-6-bromothiochroman in THF was gradually added at room temperature. The reaction mixture was refluxed for 3 hours and then cooled to 10°C, and carbon dioxide gas was bubbled for 1 hour. A 5 % hydrochloric acid aqueous solution was added to the reaction mixture, and the aqueous layer was removed. The organic layer was extracted with a 5 % potassium carbonate aqueous solution, and adjusted to a pH of 1 by adding 5 % hydrochloric acid, and a formed solid was recovered by filtration to give 7.4 g (yield 88 %) of the subject end product.
¹H-NMR (ppm, solvent: CDCl₃, internal standard: tetramethylsilane): 1.50 (6H,m), 1.90 ∼ 2.10 (2H,m), 2.25 (3H,s), 2.70 (3H,s), 2.90 ∼ 3.10 (2H,m), 7.55(1H,s)

### (1-4) Synthesis of 4,4,5,8-tetramethylthiochroman-1,1-dioxide-6-carboxylic acid

5 ml of acetic acid was added to 5 g (0.02 mol) of 4,4,5,8-tetramethylthiochroman-6-carboxylic acid, further, 6.9 g (0.06 mol) of a 30 % hydrogen peroxide aqueous solution was added, and the mixture was refluxed at 80°C for 2 hours. To the reaction mixture was added 50 ml of a 2 % sodium hydrogensulfite aqueous solution, and a solid precipitate was recovered by filtration to give 5.1 g (yield 90 %) of the subject end product.
¹H-NMR (ppm, solvent: CDCl₃, internal standard: tetramethylsilane): 1.60 (6H,s), 2.25 ∼ 2.50 (2H,m), 2.65 (3H,s), 2.75 (3H,s), 3.30 ∼ 3.50 (2H,m), 7.55(1H,s)

### B. Referential Preparation Example of Starting Material (II) Referential Preparation Example 2:

### Synthesis of Compound (V) from Carboxylic Acid (III)

(2-1) 3 ml of thionyl chloride was added to 2.77 g of 4-methoxy-5,8-dimethylthiochroman-1,1-dioxide-6-carboxylic acid corresponding to carboxylic acid (IIIa), which had been prepared in advance by a known method, and the mixture was refluxed under heat for 30 minutes. After the reaction, excessive thionyl chloride was distilled off under reduced pressure. To the remaining oil was added 10 ml of THF, to give a solution of carboxylic acid chloride in THF. On the other hand, a THF solution containing 2.0 g of tert-butyl 3-cyclopropyl-3-oxopropionate and 1.24 g of magnesium ethoxide was prepared, and the solution was refluxed under heat for 2 hours. To the resultant mixture was added the above solution of carboxylic acid chloride in THF, and the mixture was further refluxed under heat for 3 hours. After the reaction, the THF was distilled off under reduced pressure, ethyl acetate was added, and the mixture was washed with a 5 % hydrochloric acid aqueous solution and then with a saturated sodium chloride aqueous solution. The organic layer was dried over anhydrous sodium sulfate, and the solvent was distilled off under reduced pressure to give 4.4 g of tert-butyl 3-cyclopropyl-2-(4-methoxy-5,8-dimethylthiochroman-1,1-dioxide-6-carbonyl)-3-oxopropionate (Compound No. V-1) in the form of a crude brown glass-like substance.
(2-2) Compound No. V-2 was obtained in the same manner as in the above (2-1) except that the carboxylic acid used in the above (2-1) was replaced with 4,4,5,8-tetramethylthiochroman-1,1-dioxide-6-carboxylic acid corresponding to carboxylic acid (IIIc), which had been prepared in Referential Preparation Example 1, and that the diketone compound was replaced with tert-butyl 3-oxobutanoic acid.
(2-3) Compound No. V-3 was obtained in the same manner as in the above (2-1) except that the carboxylic acid used in the above (2-1) was replaced with 4,4,5,8-tetramethylthiochroman-1,1-dioxide-6-carboxylic acid corresponding to carboxylic acid (IIIc), which had been prepared in Referential Preparation Example 1.
(2-4) Compound No. V-4 was obtained in the same manner as in the above (2-1) except that the carboxylic acid used in the above (2-1) was replaced with 4-methoxy-5-methylthiochroman-1,1-dioxide-6-carboxylic acid corresponding to carboxylic acid (IIIa), which had been prepared in advance according to a known method.
(2-5) Compound No. V-5 was obtained in the same manner as in the above (2-1) except that the carboxylic acid used in the above (2-1) was replaced with 4-methoxy-5-methyl-8-chlorothiochroman-1,1-dioxide-6-carboxylic acid.
(2-6) Compound No. V-6 was obtained in the same manner as in the above (2-1) except for the following. The carboxylic acid used in the above (2-1) was replaced with 1.50 g of 4-methoxyimino-5-methylthiochroman-1,1-dioxide-6-carboxylic acid, and 0.46 ml of thionyl chloride was added to a suspension containing this carboxylic acid and 4.5 ml of dichloroethane. The mixture was allowed to react at 50°C for 3.5 hours. After the reaction, the solvent and excessive thionyl chloride were distilled off under reduced pressure, and 4 ml of THF was added to the residue to give a THF solution of carboxylic acid chloride.
(2-7) Compound No. V-7 was obtained in the same manner as in the above (2-6) except that the carboxylic acid used in the above (2-6) was replaced with 4-methoxyimino-5,8-dimethylthiochroman-1,1-dioxide-6-carboxylic acid.
(2-8) Compound No. V-8 was obtained in the same manner as in the above (2-6) except that the carboxylic acid used in the above (2-6) was replaced with 4-methoxyimino-5,8-dimethylthiochroman-6-carboxylic acid.
(2-9) Compound No. V-9 was obtained in the same manner as in the above (2-6) except that the carboxylic acid used in the above (2-6) was replaced with 4-propargyloxyimino-5,8-dimethylthiochroman-1,1-dioxide-6-carboxylic acid.
(2-10) Compound No. V-10 was obtained in the same manner as in the above (2-6) except that the carboxylic acid used in the above (2-6) was replaced with 4-allyloxyimino-5,8-dimethylthiochroman-1,1-dioxide-6-carboxylic acid.
(2-11) Compound No. V-11 was obtained in the same manner as in the above (2-6) except that the carboxylic acid used in the above (2-6) was replaced with 4-(2-fluoroethoxy)imino-5,8-dimethylthiochroman-1,1-dioxide-6-carboxylic acid.
(2-12) Compound No. V-12 was obtained in the same manner as in the above (2-6) except that the carboxylic acid used in the above (2-6) was replaced with 4-(2-fluoroethoxy)-5,8-dimethylthiochroman-1,1-dioxide-6-carboxylic acid.
(2-13) Compound No. V-13 was obtained in the same manner as in the above (2-6) except that the carboxylic acid used in the above (2-6) was replaced with 4-allyloxy-5,8-dimethylthiochroman-1,1-dioxide-6-carboxylic acid.
(2-14) Compound No. V-14 was obtained in the same manner as in the above (2-1) except that the carboxylic acid used in the above (2-1) was replaced with 3,3,5-trimethylthiochroman-4-one-1,1-dioxide-6-carboxylic acid.
(2-15) Compound No. V-15 was obtained in the same manner as in the above (2-1) except that the carboxylic acid used in the above (2-1) was replaced with 3,3,5,8-tetramethylthiochroman-4-one-1,1-dioxide-6-carboxylic acid.

Table 1 shows the structural formulae of the compounds obtained in the above (2-1) to (2-15).

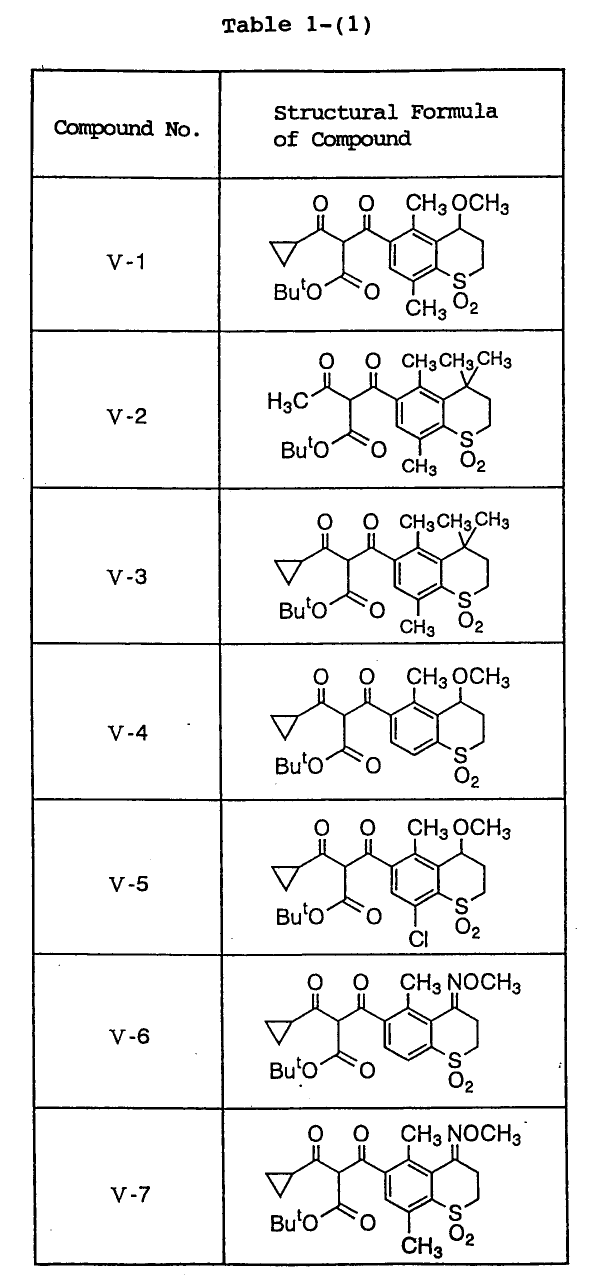

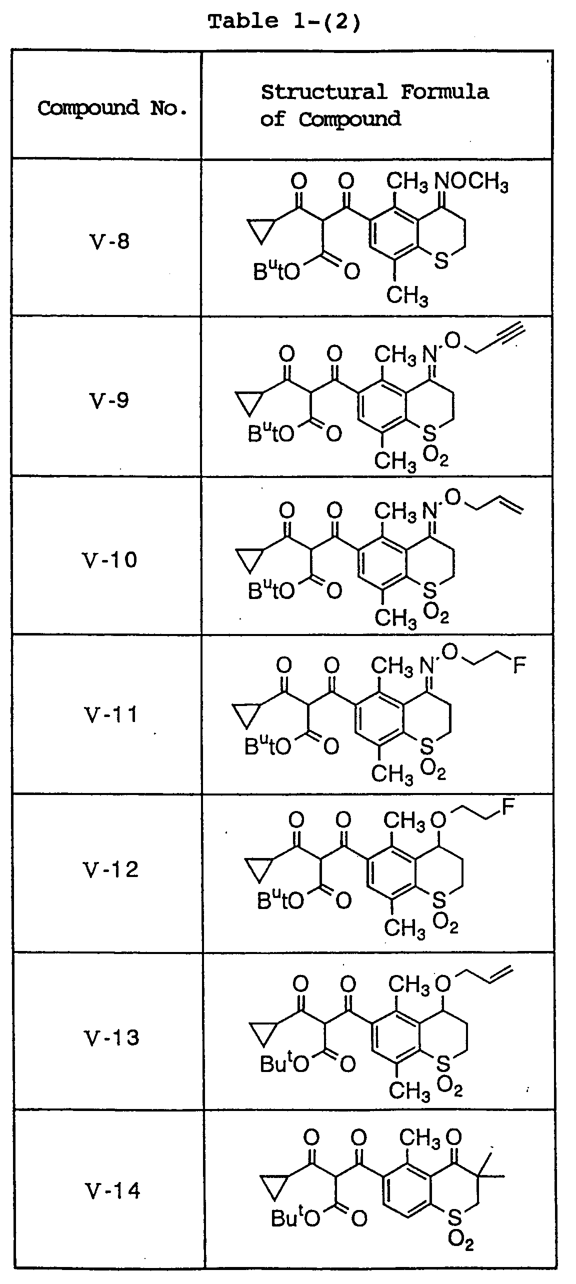

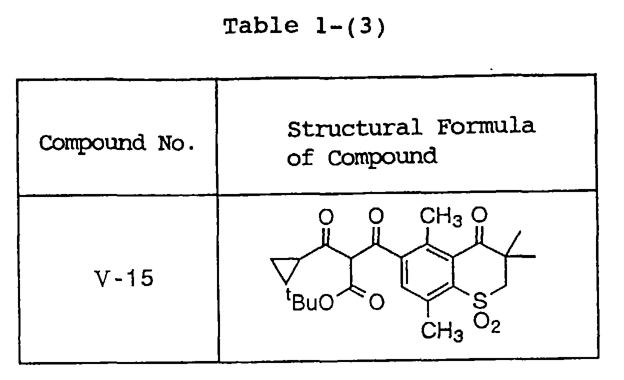

### Referential Preparation Example 3-A:

### Synthesis of diketone intermediate (VII) from Compound (V)

(3-1) A mixture containing 4.3 g of tert-butyl 3-cyclopropyl-2-(4-methoxy-5,8-dimethylthiochroman-1,1-dioxide-6-carbonyl)-3-oxopropionate (Compound No. V-1) synthesized in the above (2-1), 0.20 g of 4-toluenesulfonic acid and 40 ml of toluene was refluxed under heat for 3 hours. The reaction mixture was cooled, and then ethyl acetate was added. The mixture was dried over sodium carbonate and then filtered. The filtrate was subjected to evaporation under reduced pressure to remove the solvent, and the resultant residue was purified by flash column chromatography (Wakogel C-300: hexane/ethyl acetate = 2:1) to give 2.63 g of 1-cyclopropyl-3-(4-methoxy-5,8-dimethylthiochroman-1,1-dioxide-6-yl)propan-1,3-dione (Compound No. VII-1) in the form of a white glass-like substance.
(3-2) ∼ (3-15) Compounds Nos. VII-2 ∼ VII-15 were obtained in the same manner as in the above (3-1) except that the starting material in the (3-1) was replaced with Compounds Nos. V-2 ∼ V-15 obtained in the above (2-2) ∼ (2-15).

Table 2 shows structural formulae and NMR data of the Compounds VII-2 ∼ VII-15 obtained in the above (3-1) ∼ (3-15).

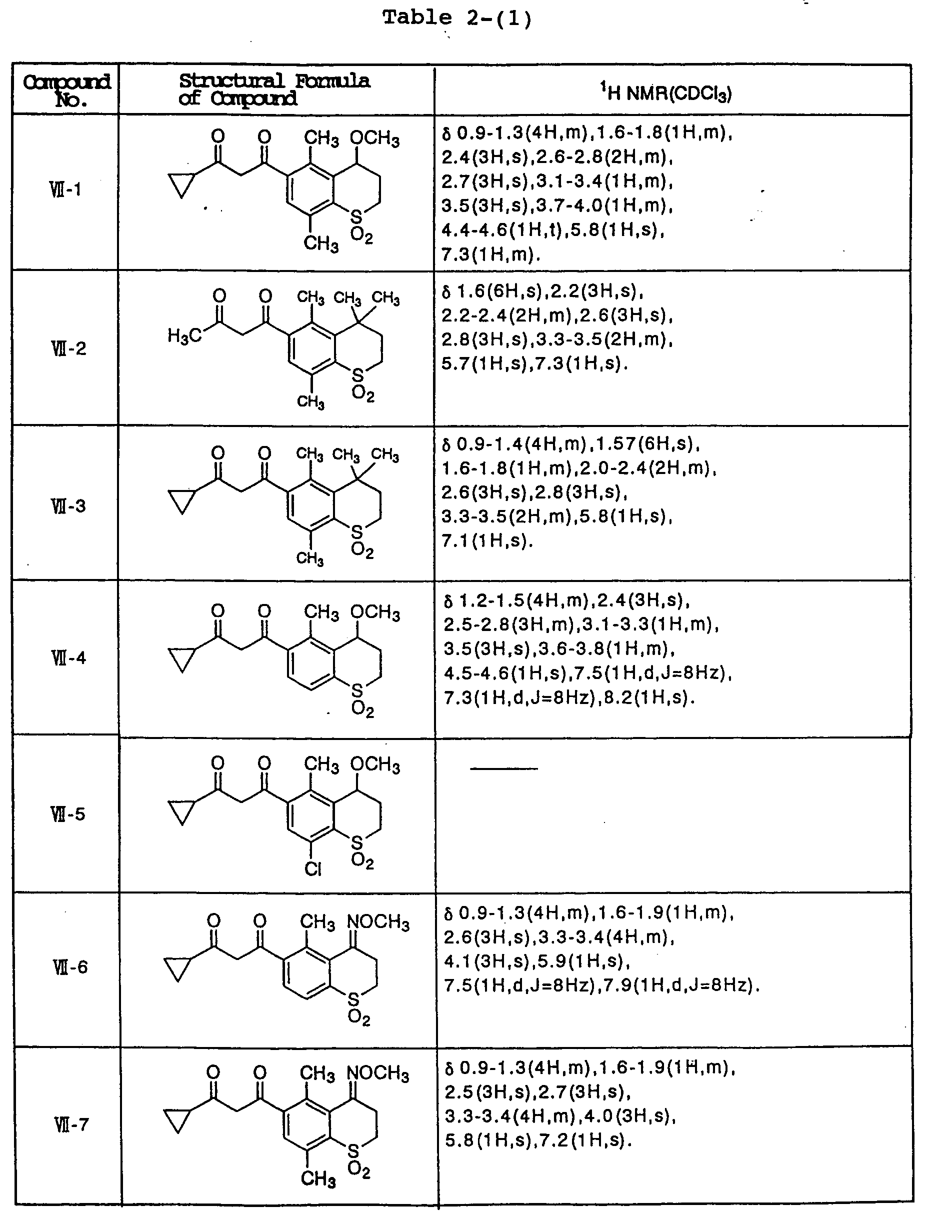

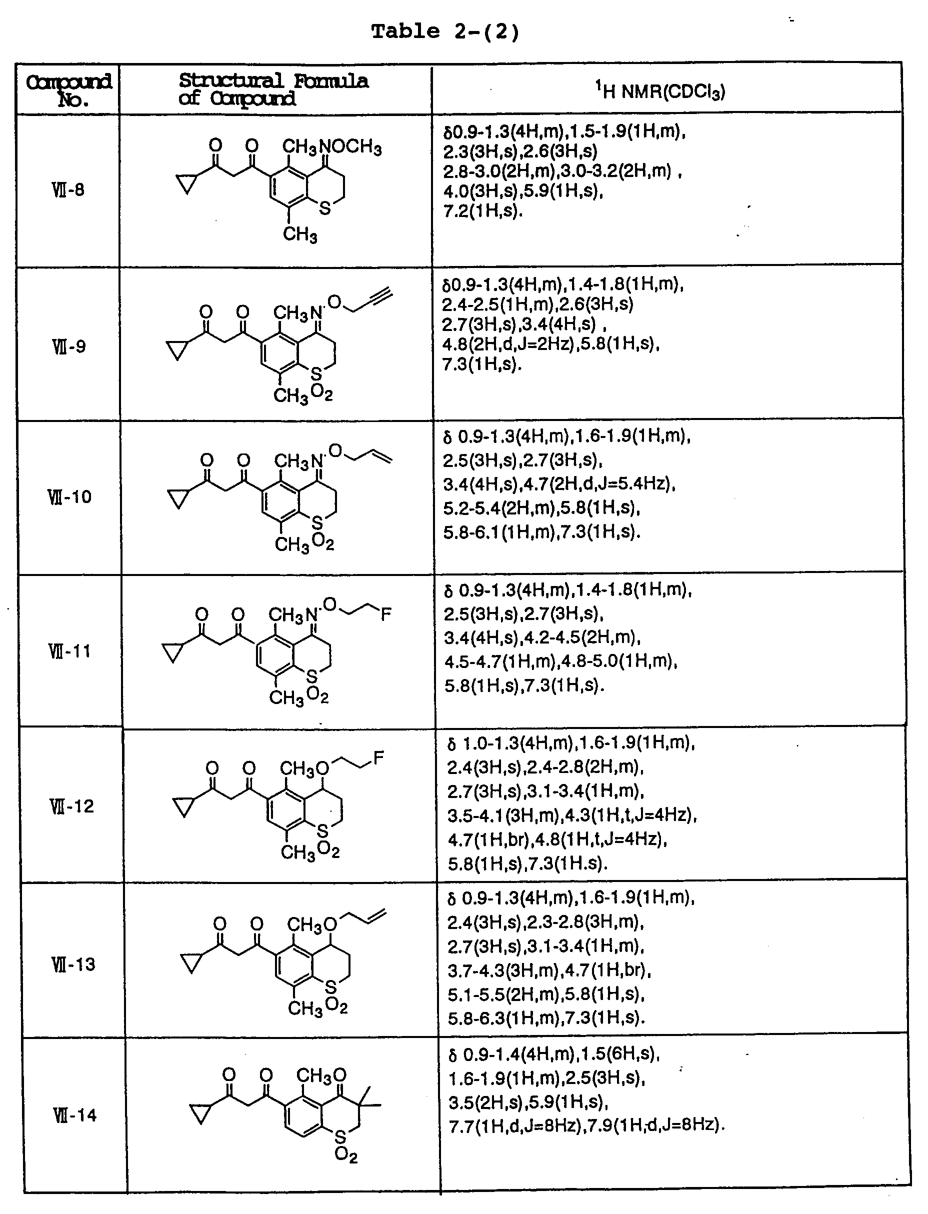

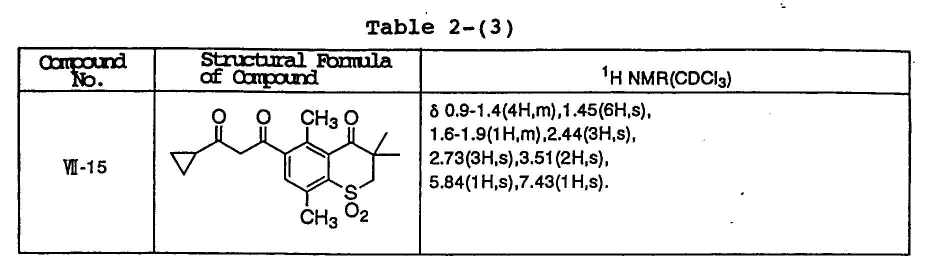

### Referential Preparation Example 3-B:

### Synthesis of diketone intermediate (VII) from carboxylic acid ester derivative (III')

(3-16) A tetrahydrofuran solution containing 1.00 g of ethyl 4,4-dioxyethylene-5,8-dimethylthiochroman-1,1-dioxide-6-carboxylate and 0.49 g of cyclopropyl methyl ketone was dropwise added to a suspension of sodium hydride (0.26 g of 60 % sodium hydride in oil) in tetrahydrofuran under reflux under heat. After the completion of the addition, the mixture was stirred under reflux under heat for 3 hours. Further, 0.26 g of 60 % sodium hydride in oil was added, and the mixture was stirred under reflux under heat for 6 hours. Then, the reaction mixture was cooled to room temperature, water was gradually added, and a 5 % hydrochloric acid aqueous solution was added to adjust the mixture to a pH of 1. The mixture was extracted with ethyl acetate, and the extract was washed with a saturated sodium hydrogencarbonate aqueous solution and then with a saturated sodium chloride aqueous solution and dried over anhydrous sodium sulfate. The resultant product was filtered, and then the solvent was distilled off under reduced pressure. The residue was purified by flash column chromatography (silica gel; hexane/ethyl acetate = 1:2) to give 0.70 g of 3-cyclopropyl-1-(4,4-dioxyethylene-5,8-dimethylthiochroman-1,1-dioxide-6-yl)propan-1,3-dione (Compound No VII-16) in the form of a white solid.
(3-17) A tetrahydrofuran solution containing 1.80 g of ethyl 4,4-dioxyethylene-5,8-dimethylthiochroman-6-carboxylate and 0.99 g of cyclopropyl methyl ketone was dropwise added to a suspension of sodium hydride (0.52 g of 60 % sodium hydride in oil) in tetrahydrofuran under reflux under heat. After the completion of the addition, the mixture was stirred under reflux under heat for 6 hours. Further, 0.52 g of 60 % sodium hydride in oil was added, and the mixture was stirred under reflux under heat for 3 hours. Then, the reaction mixture was cooled to room temperature, water was gradually added, and a 5 % hydrochloric acid aqueous solution was added to adjust the mixture to a pH of 1. The mixture was extracted with ethyl acetate, and the extract was washed with a saturated sodium hydrogencarbonate aqueous solution and then with a saturated sodium chloride aqueous solution and dried over anhydrous sodium sulfate. The resultant product was filtered, and then the solvent was distilled off under reduced pressure. The residue was purified by flash column chromatography (silica gel; hexane/ethyl acetate = 3:1) to give 0.63 g of 3-cyclopropyl-1-(4,4-dioxyethylene-5,8-dimethylthiochroman-6-yl)propan-1,3-dione (Compound No. VII-17) in the form of a yellow oil.

Table 3 shows structural formulae and NMR data of the compounds obtained in the above (3-16) ∼ (3-17).

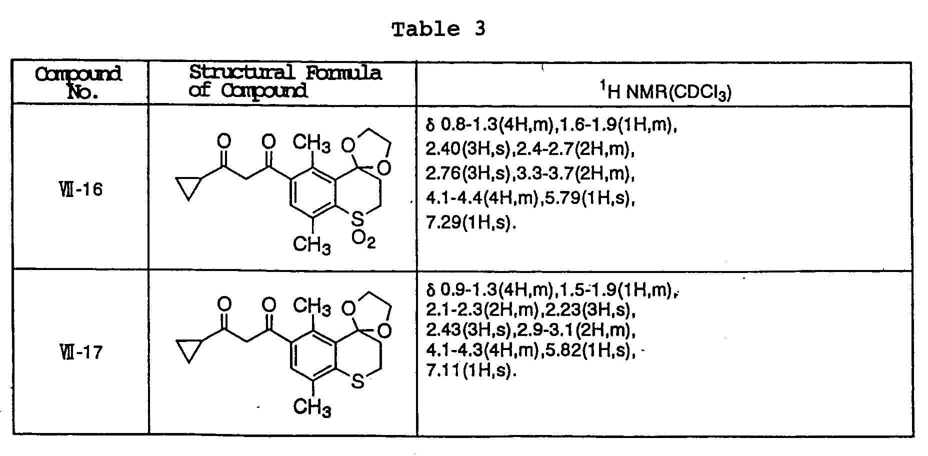

### Referential Preparation Example 4:

### Synthesis of starting material (II) from diketone intermediate (VII)

(4-1) 2.63 g of the 1-cyclopropyl-3-(4-methoxy-5,8-dimethylthiochroman-1,1-dioxide-6-yl)propane-1,3-dione (Compound No. VII-1) synthesized in the above (3-1) and 1.60 ml of dimethylformamide were added to 15 ml of dioxane, and the mixture was stirred at room temperature for 4 days. After the reaction, the solvent was distilled off to give 2.84 g of 3-cyclopropyl-2-(dimethylamino)methylidene-1-(4-methoxy-5,8-dimethylthiochroman-1,1-dioxide-6-yl)-1,3-dione (Compound No. II-1) in the form of an orange solid.
(4-2) ∼ (4-17) Compounds Nos. II-2 ∼ II-17 were obtained in the same manner as in the above (4-1) except that the diketone intermediate used in the (4-1) was replaced with Compounds Nos. VII-2 ∼ VII-17 obtained in the above (3-2) ∼ (3-17).

Table 4 shows structural formulae and NMR data of the compounds obtained in the above (4-1) ∼ (4-17).

The compounds obtained in the above (4-1) ∼ (4-17) were used for the synthesis of isoxazole derivatives (I) of the present invention without purification.

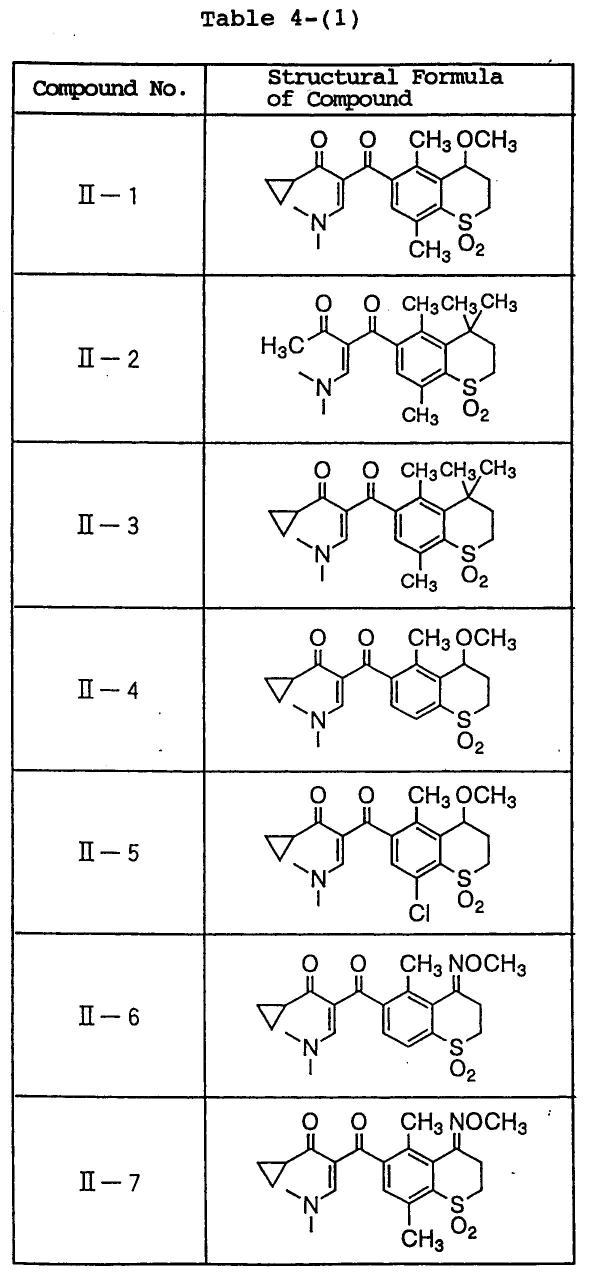

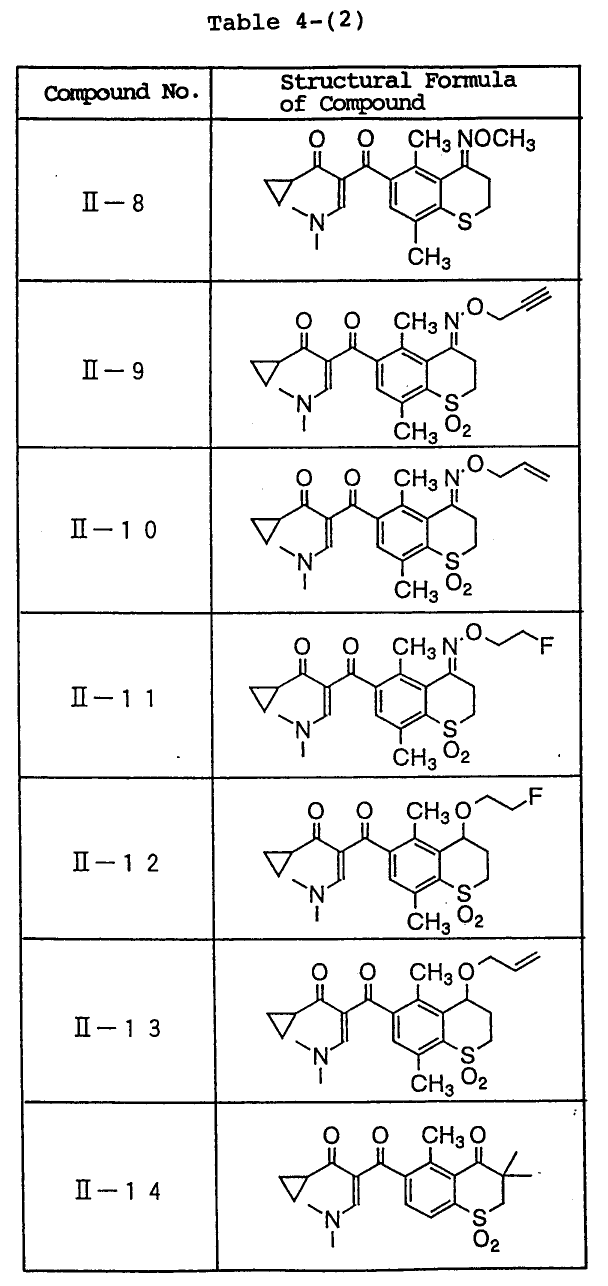

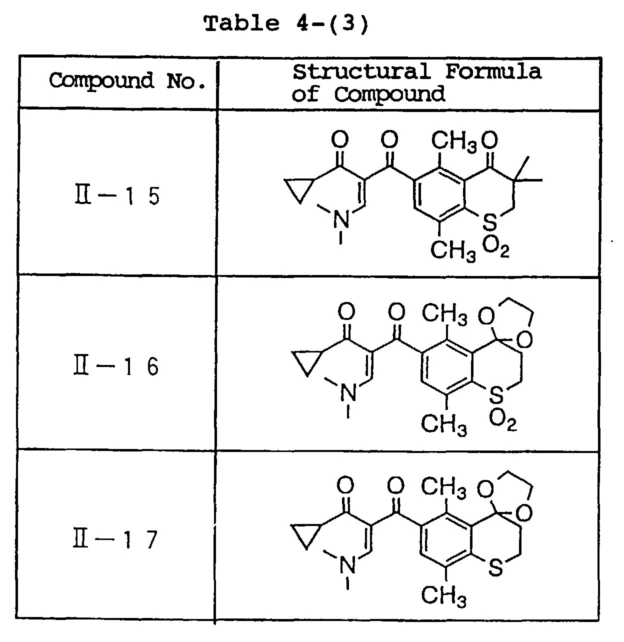

### C. Preparation Example of Isoxazole Derivative (I)

### Preparation Example 1

1.60 g of the crude 3-cyclopropyl-2-(dimethylamino)methylidene-1-(4-methoxy-5,8-dimethylthiochroman-1,1-dioxide-6-yl)propane-1,3-dione (Compound No. II-1) synthesized in the above (4-1) and 0.32 g of hydroxylamine hydrochloride were added to ethanol, and the mixture was stirred at room temperature overnight. After the reaction, the solvent was distilled off, and the residue was dissolved in methylene chloride. The resultant solution was washed with water, and an organic layer was dried over anhydrous sodium sulfate. The solvent was distilled off under reduced pressure, and the residue was purified by flash column chromatography (Wakogel C-300; hexane/ethyl acetate = 2:1) to give 1.30 g of 5-cyclopropyl-4-(4-methoxy-5,8-dimethylthiochroman-1,1-dioxide-6-carbonyl)isoxazole (Compound No. 1) in the form of a white glass-like substance, which was the intended isoxazole derivative included in the general formula (I).

### Preparation Examples 2 - 17

Compounds Nos. 2∼17 were obtained in the same manner as in Preparation Example 1 except that the starting material used in Preparation Example 1 was replaced with Compounds Nos. II-2 ∼ II-17 synthesized in the above (4-2) ∼ (4-17).

Table 5 shows the structural formulae of Compounds Nos. 1 ∼ 17 obtained in Preparation Examples 1 ∼ 17. Table 6 shows the NMR data, IR data and melting points of Compounds 1 ∼ 17.

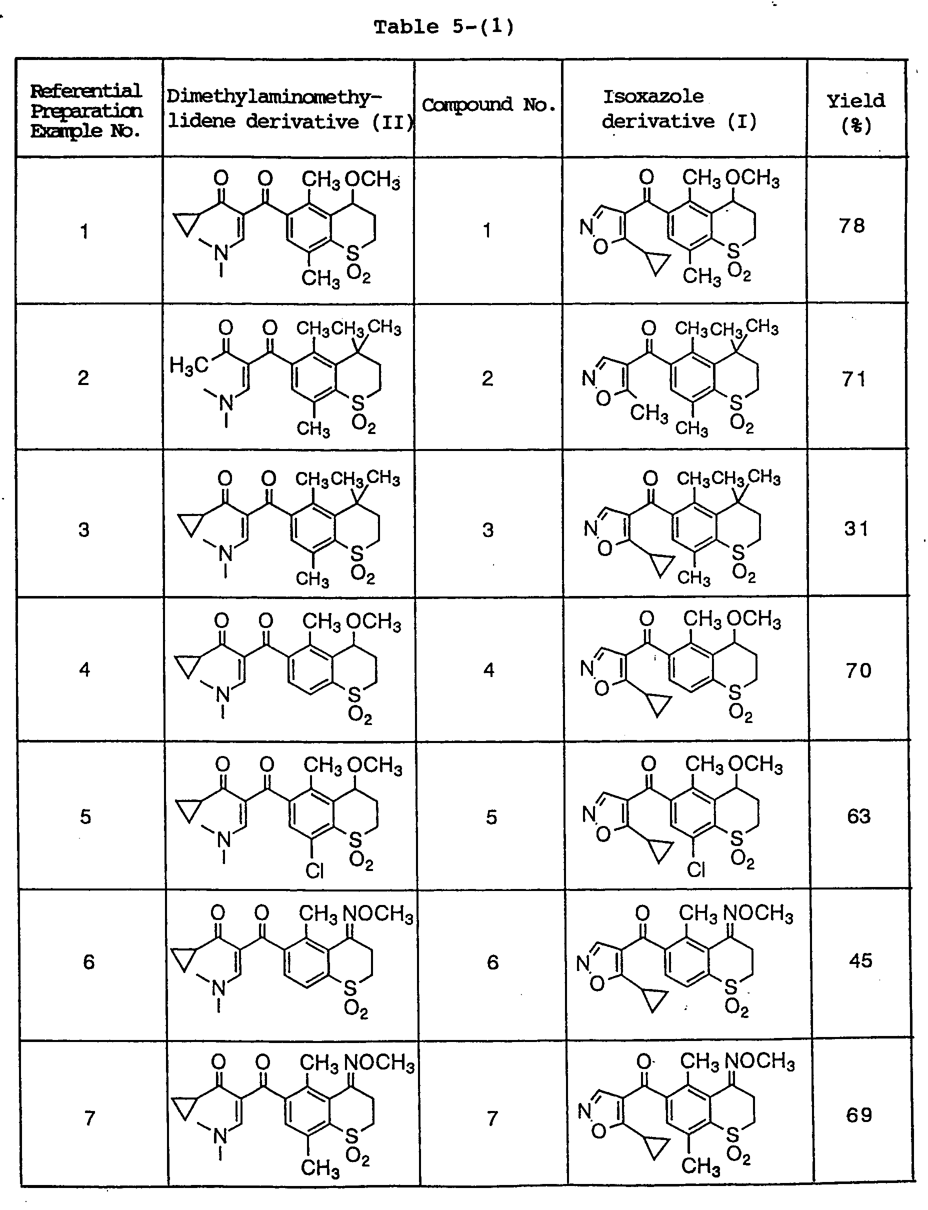

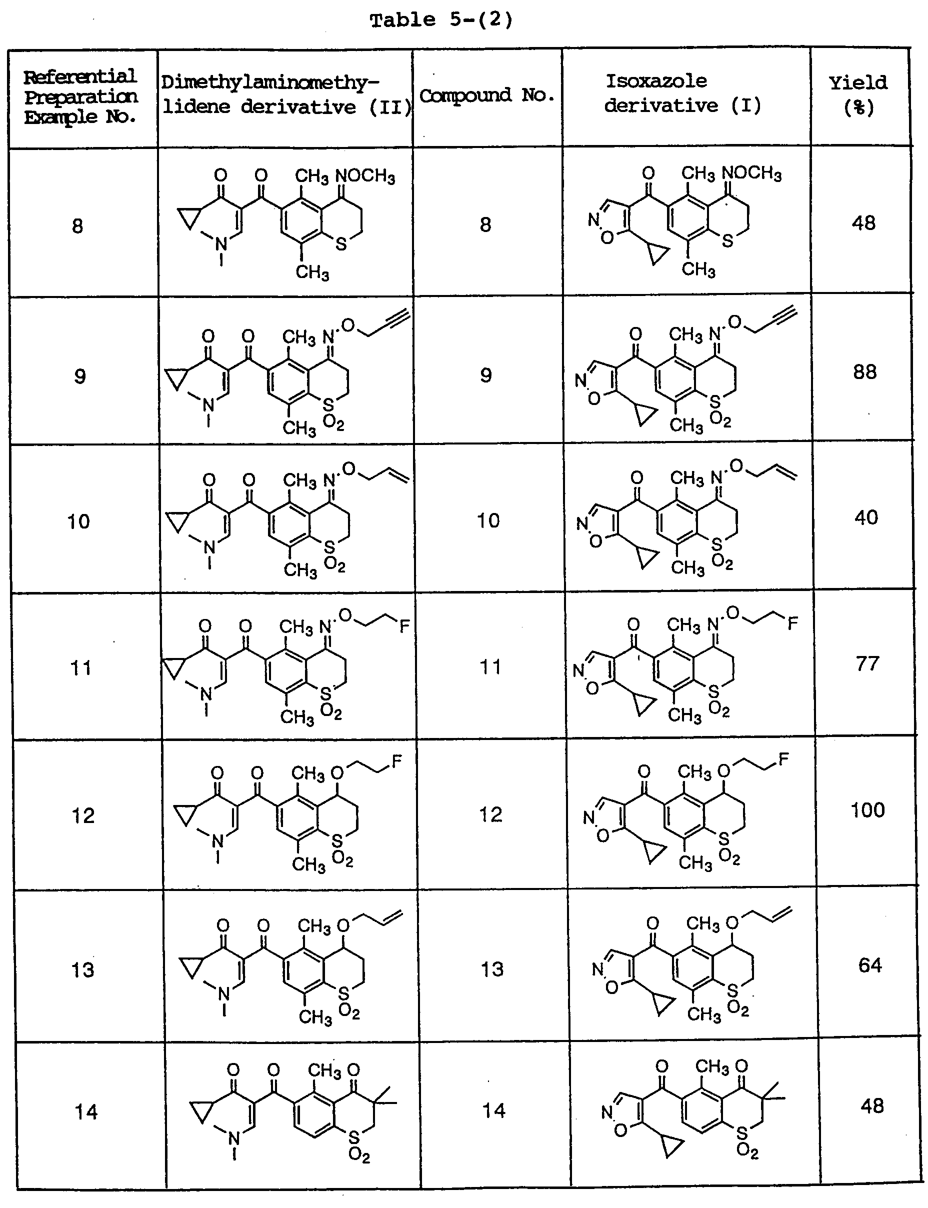

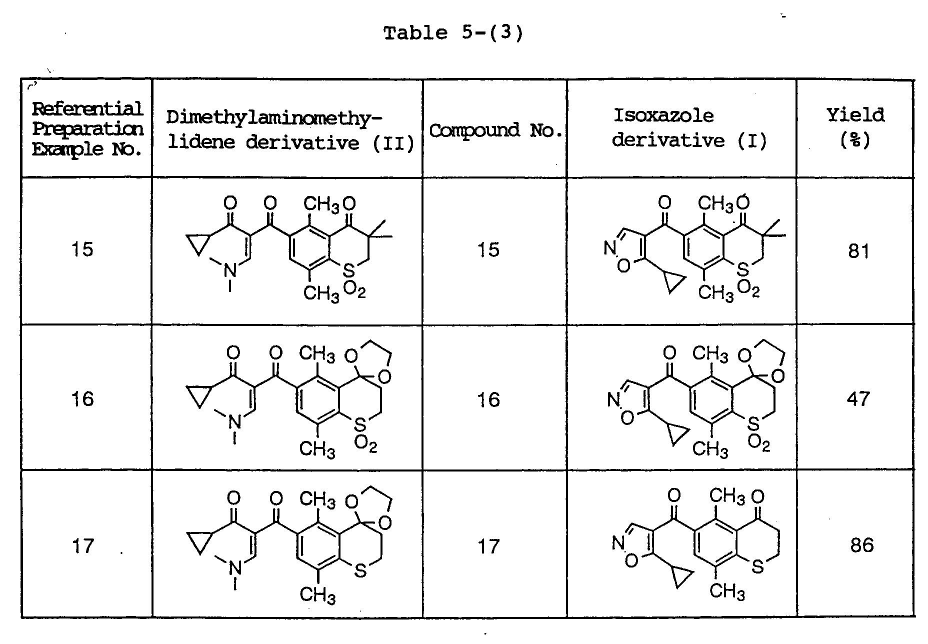

**Table 6-(1)**

| Compound No. | ¹H NMR (ppm) Internal Standard : Tetramethylsilane Solvent : CDCl₃ | IR (cm⁻¹) | Melting point (°C) |
|---|---|---|---|
| 1 | δ 1.2-1.4(4H,m),2.34(3H,m), | 2960 | Glass-like substance |
| | 2.5-2.7(3H,m),2.76(3H,s), | 1680 | |
| | 3.1-3.4(1H,m),3.47(3H,s), | 1300 | |
| | 3.6-4.1(1H,m)4.53(1H,m) | 1140 | |
| | 7.23(1H,s),8.17(1H,s). | | |
| 2 | δ 1.58(6H,s),2.3-2.5(2H,m), | 2960 | Glass-like substance |
| | 2.45(3H,s),2.60(3H,s), | 2670 | |
| | 2.79(3H,s),3.3-3.5(2H,m), | 1300 | |
| | 7.03(1H,s),8.22(1H,s). | 1140 | |
| 3 | δ 1.2-1.4(4H,m),1.56(6H,s), | 2960 | Glass-like substance |
| | 2.3-2.5(2H,m),2.48(3H,s), | 1680 | |
| | 2.5-2.7(1H,m),2.79(3H,s), | 1290 | |
| | 3.3-3.5(2H,m),7.07(1H,s), | 1130 | |
| | 8.15(1H,s). | | |
| 4 | δ 1.2-1.5(4H,m),2.39(3H,s), | 2970 | Glass-like substance |
| | 2.5-2.8(3H,m),3.1-3.3(1H,m), | 1680 | |
| | 3.49(3H,s),3.6-3.8(1H,m), | 1300 | |
| | 4.5-4.6(1H,t),7.49(1H,d,J=8.1Hz), | 1140 | |
| | 7.92(1H,d,J=8.1Hz),8.15(1H,s). | | |
| 5 | δ 1.2-1.5(4H,m),2.3(3H,s), | 2970 | Glass-like substance |
| | 2.5-2.8(3H,m),3.1-3.3(1H.m), | 1580 | |
| | 3.5(3H,s),3.7-4.1(1H,m), | 1330 | |
| | 4.5(1H,t),7.5(1H,s), | 1140 | |
| | 8.2(1H,s). | | |

**Table 6-(2)**

| Compound No. | ¹H NMR (ppm) Internal Standard : Tetramethylsilane Solvent : CDCl₃ | IR (cm⁻¹) | Melting point (°C) |
|---|---|---|---|
| 6 | δ 1.2-1.4(4H,m),2.5-2.8(1H,m), | 2970 | Glass-like substance |
| | 2.6(3H,s),3.4(4H,br), | 1680 | |
| | 4.1(3H,s),7.5(1H,d,J=8Hz), | 1330 | |
| | 8.0(H,d,J=8Hz),8.2(1H,s). | 1140 | |
| 7 | δ 1.2-1.4(4H,m),2.4-2.8(1H,m), | 2960 | Glass-like substance |
| | 2.5(3H,s),2.7(3H,s), | 1670 | |
| | 3.3(4H,br),4.0(3H,s), | 1320 | |
| | 7.2(1H,s),8.2(1H,s). | 1150 | |
| 8 | δ 1.1-1.4(4H,m),2.3(3H,s), | 2960 | 86.2-88.2 |
| | 2.5(3H,s),2.4-2.7(1H,m), | 1675 | |
| | 2.8-3.0(2H,m),3.0-3.2(2H,m), | 1060 | |
| | 4.0(3H,s),7.1(1H,s), | | |
| | 8.3(1H,s). | | |
| 9 | δ 1.2-1.5(4H,m),2.5(3H,s), | 2950 | 144.2-145.0 |
| | 2.4-2.7(2H,m),2.7(3H,s), | 1670 | |
| | 3.4(3H,s),4.8(2H,d,J=3Hz). | 1320 | |
| | 7.2(1H,s),8.2(1H,s). | 1135 | |
| 10 | δ 1.2-1.5(4H,m),2.4(3H,s), | 2940 | 152.5-154.0 |
| | 2.4-2.7(1H,m),2.7(3H,s), | 1675 | |
| | 4.0(4H,s),4.7(2H,d,J=5Hz), | 1320 | |
| | 5.2-5.4(3H,m),5.8-6.3(1H,m), | 1130 | |
| | 7.2(1H,s),8.2(1H,s). | | |

**Table 6-(3)**

| Compound No. | ¹H NMR (ppm) Internal Standard : Tetramethylsilane Solvent : CDCl₃ | IR (cm⁻¹) | Melting point (°C) |
|---|---|---|---|
| 11 | δ 1.2-1.5(4H,m),2.4(3H,s), | 2955 | 158.1-162.0 |
| | 2.4-2.8(1H,m),2.7(3H,s), | 1675 | |
| | 3.4(4H,s),4.2-4.5(2H,m), | 1320 | |
| | 4.5-4.7(1H,m),4.8-5.0(1H,m), | 1130 | |
| | 7.2(1H,s),8.2(1H,s). | | |
| 12 | δ 1.2-1.5(4H,m),2.4(3H,s), | 2960 | 64-74 |
| | 2.3-2.8(3H,m),2.8(3H,s), | 1680 | |
| | 3.1-3.4(1H,m),3.6-4.1(3H,m), | 1300 | |
| | 4.3(1H,t),4.7(1H,br), | 1140 | |
| | 4.8(1H,t),7.3(1H,s).8.2(1H,s). | | |
| 13 | δ1.2-1.4(4H,m),2.4(3H,s), | 2950 | 62-63 |
| | 2.3-2.8(3H,m),2.8(3H,s), | 1670 | |
| | 3.1-3.4(1H,m),3.7-4.3(3H,m), | 1295 | |
| | 4.7(1H,br),5.1-5.5(2H,m), | 1130 | |
| | 5.7-6.2(1H,m),7.2(1H,s),8.2(1H,s). | | |
| 14 | δ1.2-1.5(4H,m),1.5(6H,s), | 3000 | Glass-like substance |
| | 2.4-2.7(1H,s),2.5(3H,s), | 1715 | |
| | 3.5(2H,s),7.6(1H,d,J=8Hz), | 1675 | |
| | 7.8.0(1H,d,J=8Hz),8.2(1H,s). | 1325 | |
| | | 1135 | |
| 15 | δ1.2-1.6(4H,m),1.5(6H,s), | 3000 | 124.2-131.2 |
| | 2.4(3H,s),2.4-2.8(1H,m), | 1700 | |
| | 2.8(3H,s),3.6(2H,s), | 1675 | |
| | 7.4(1H,s),8.2(1H,s). | 1320 | |
| | | 1135 | |

**Table 6-(4)**

| Compound No. | ¹H NMR (ppm) Internal Standard : Tetramethylsilane Solvent : CDCl₃ | IR (cm⁻¹) | Melting point (°C) |
|---|---|---|---|
| 16 | δ1.1-1.4(4H,m),2.3(3H,s), | 2940 | 157.1-159.0 |
| | 2.4-2.7(3H,m),2.7(3H,s), | 1680 | |
| | 3.4-3.7(2H,m),4.1-4.4(4H,m), | 1300 | |
| | 7.2(1H,s),8.2(1H,s). | 1120 | |
| 17 | δ1.1-1.4(4H,m),2.3(3H,s), | 2950 | 138-141 |
| | 2.5(3H,s),2.4-2.8(1H,m), | 1680 | |
| | 2.9-3.4(4H,m),7.2(1H,s), | 1660 | |
| | 8.2(1H,s). | | |

The herbicide of the present invention will be further explained specifically with reference to Herbicide Examples and Herbicide Comparative Examples hereinafter.

### Herbicide Examples and Herbicide Comparative Examples

### (1) Preparation of Herbicide

97 Parts by weight of talc (trade name: Zeaklite) as a carrier, 1.5 parts by weight of alkylarylsulfonic acid (trade name: Neoplex, supplied by Kao-Atlas K.K.) as a surfactant and 1.5 parts by weight of a nonionic and anionic surfactant (trade name: Sorpol 800A, supplied by Toho Chemical Co., Ltd.) were uniformly pulverized and mixed to prepare a carrier for a wettable powder.

90 Parts by weight of the above carrier for a wettable powder and 10 parts by weight of one of the compounds of the of the present invention obtained in the above Preparation Examples 1 ∼ 17 (or 10 parts by weight of one of the following compounds (A) and (B) for Comparative Examples) were uniformly pulverized and mixed to obtain a herbicide.

The following compound (A) for Comparative Example is disclosed in WO93/18031, and the following compound (B) for Comparative Example is disclosed in EP95/0636622. The compounds (A) and (B) have the following structures.

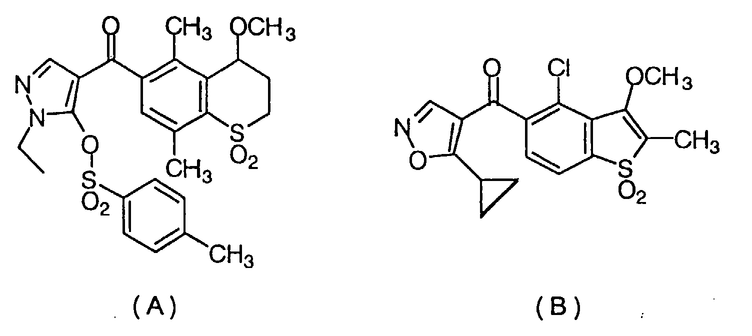

The herbicidal efficacy and phytotoxicity to crops were shown on the basis of the following ratings.

### (Ratings)

| Herbicidal efficacy | Ratio of remaining plant weight to non-treated (%) |
|---|---|
| 0 | 81 - 100 |
| 1 | 61 - 80 |
| 2 | 41 - 60 |
| 3 | 21 - 40 |
| 4 | 1 - 20 |
| 5 | 0 |

| Phytotoxicity to crops | Ratio of remaining plant weight to non-treated (%) |
|---|---|
| - | 100 |
| ± | 95 - 99 |
| + | 90 - 94 |
| ++ | 80 - 89 |
| +++ | 0 - 79 |

The above ratio of remaining plant weight to non-treated was determined on the basis of the ratio of remaining plant weight to non-treated = (remaining plant weight in treated plot/remaining plant weight in non-treated plot) x 100.

### (2) Soil treatment test on upland soil 1

Seeds of weeds such as velvetleaf, pale smart weed, jimsonweed, smooth amaranth, black nightshade, barnyardgrass and large crabgrass and seeds of corn and cotton were sown in 1/5,000-are Wagner pots filled with upland soil, and covered with upland soil. Then, a predetermined amount of the herbicide prepared in the above (1) was suspended in water and uniformly sprayed onto the soil surface. Thereafter, the seeds were grown in a greenhouse, and on 20th day after the treatment, the herbicide was evaluated for herbicidal efficacy and phytotoxicity to the crops. Table 7 shows the results.

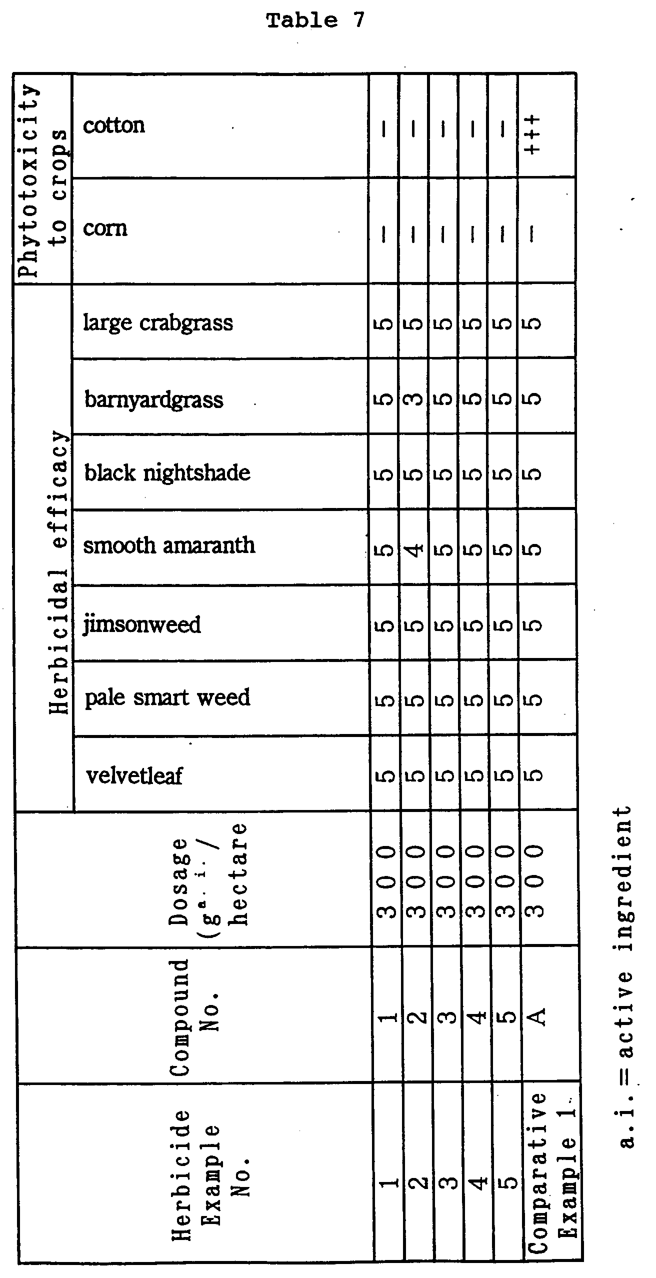

The results in Table 7 show that the herbicide of the present invention can selectively control a broad range of upland weeds at a low dosage without causing any phytotoxicity on corn and cotton. Meanwhile, it is seen that the comparative compound (A) is poor in the selectivity to cotton.

### (3) Soil treatment test on upland soil 2

Seeds of weeds such as velvetleaf, pale smart weed, jimsonweed, smooth amaranth, black nightshade, barnyardgrass and large crabgrass and seeds of corn and cotton were sown in 1/5,000-are Wagner pots filled with upland soil, and covered with upland soil. Then, a predetermined amount of the herbicide prepared in the above (1) was suspended in water and uniformly sprayed onto the soil surface. Thereafter, the seeds were grown in a greenhouse, and on 20th day after the treatment, the herbicide was evaluated for herbicidal efficacy and phytotoxicity to the crops. Table 8 shows the results.

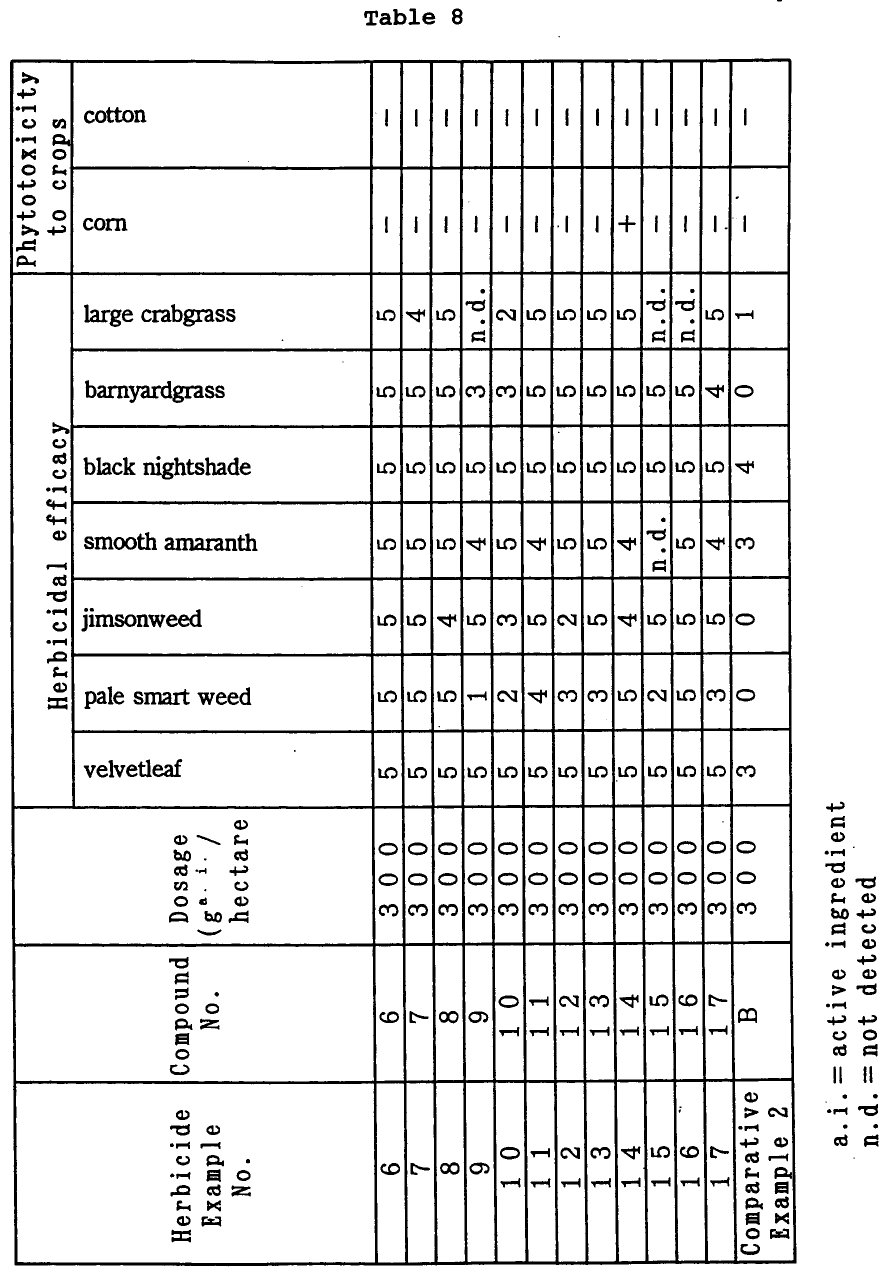

The results in Table 8 show that the herbicide of the present invention can selectively control a broad range of upland weeds at a low dosage without causing any phytotoxicity on corn and cotton. Meanwhile, it is seen that the comparative compound (B) is generally poor in the herbicidal efficacy, particularly poor in the herbicidal efficacy on pale smart weed, jimsonweed, barnyardgrass and large crabgrass.

### (4) Soil treatment test on upland soil 3 (test with winter weeds)

Seeds of weeds such as common chickweed, sentless chamomile, viola sp., persian speedwell, shepherdspurse, blackgrass and wild oat and seeds of wheat and barley were sown in 1/5,000-are Wagner pots filled with upland soil, and covered with upland soil. Then, a predetermined amount of the herbicide prepared in the above (1) was suspended in water and uniformly sprayed onto the soil surface. Thereafter, the seeds were grown in a greenhouse, and on 20th day after the treatment, the herbicide was evaluated for herbicidal efficacy and phytotoxicity to the crops. Table 9 shows the results.

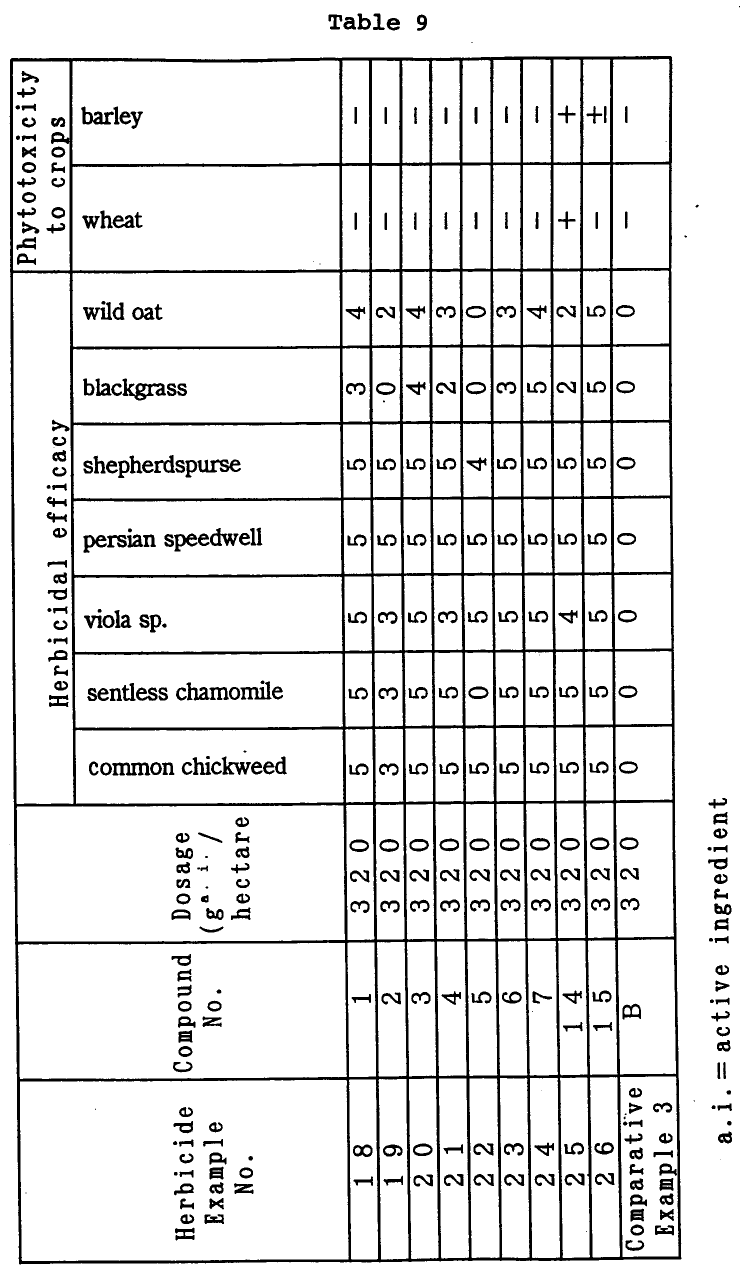

The results in Table 9 show that the herbicide of the present invention can selectively control a broad range of winter upland weeds at a low dosage without causing any phytotoxicity on wheat and barley. Meanwhile, it is seen that the comparative compound (B) is generally poor in the herbicidal efficacy.

### (5) Foliar treatment test on upland soil (test with winter weeds)

Seeds of weeds such as common chickweed, sentless chamomile, viola sp., shepherdspurse, blackgrass and wild oat and seeds of wheat and barley were sown in 1/5,000-are Wagner pots filled with upland soil, and covered with upland soil. The seeds were grown in a greenhouse, and at the stage of 3 ∼ 4 leaves of these plants, a predetermined amount of the herbicide prepared in the above (1) was suspended in water and uniformly sprayed onto leaf and stalk portions at a rate of 2,000 l/ha. Then, the plants were grown in the greenhouse, and on 30th day after the treatment, the herbicide was evaluated for herbicidal efficacy and phytotoxicity to the crops. Table 10 shows the results.

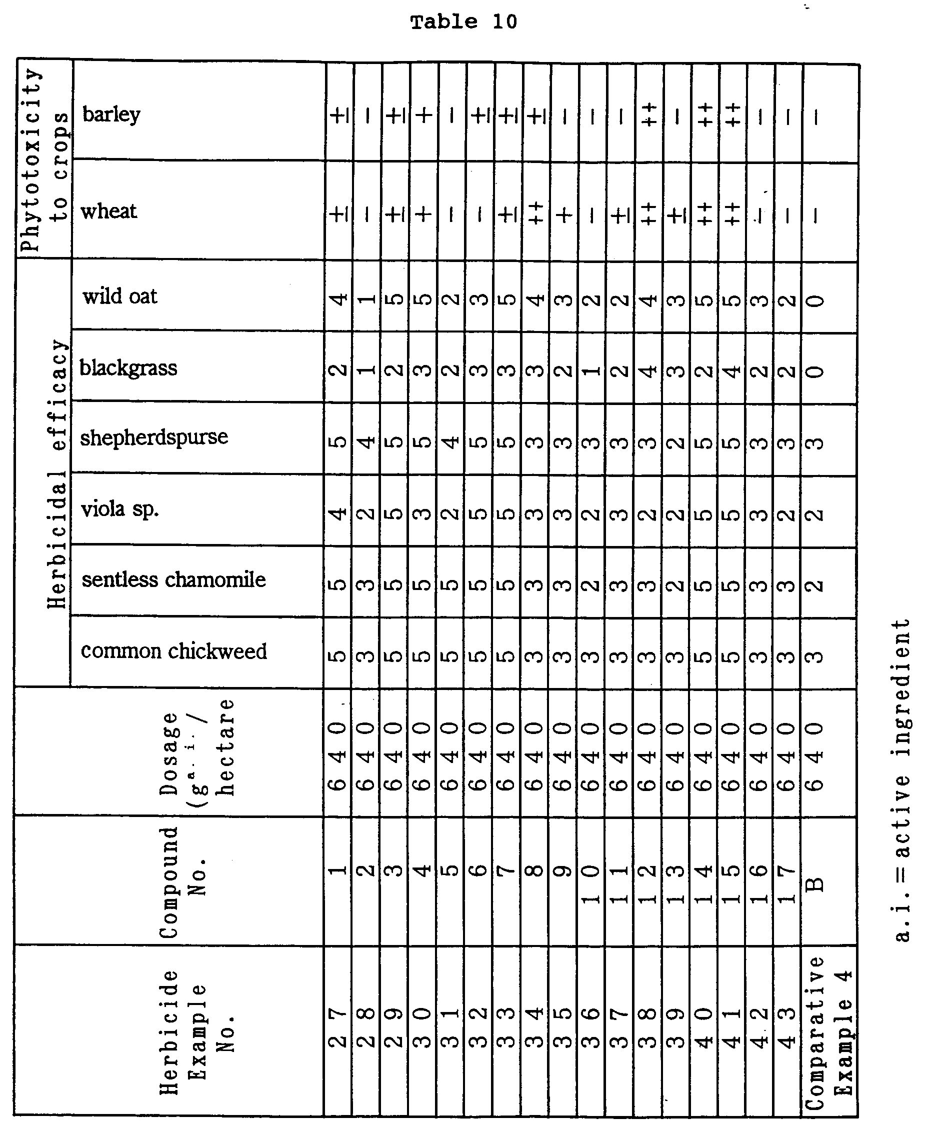

The results in Table 10 show that the herbicide of the present invention can selectively control a broad range of winter upland weeds at a low dosage without causing any phytotoxicity on wheat and barley. Meanwhile, it is seen that the comparative compound (B) is generally poor in the herbicidal efficacy, particularly poor in the herbicidal efficacy on blackgrass and wild oat.

According to the present invention, there are provided novel isoxazole derivatives and herbicides containing these as active ingredients, which can selectively control a broad range of upland soil weeds such as gramineous weeds and broad-leaved weeds without causing any phytotoxicity on useful crops such as corn and cotton.

## Claims

1. An isoxazole derivative of the general formula (I), 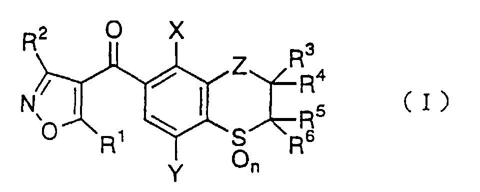 wherein:
R¹ is a C₁-C₆ alkyl group or C₃-C₆ cycloalkyl group,
R² is a hydrogen atom or a C₁∼C₄ alkoxycarbonyl group,
each of R³ ∼ R⁶ is independently a hydrogen atom or a C₁∼C₄ alkyl group,
n is an integer of 0, 1 or 2,
X is a hydrogen atom or a C₁∼C₄ alkyl group,
Y is a hydrogen atom, a C₁∼C₄ alkyl group or a halogen atom, and
Z is a group of 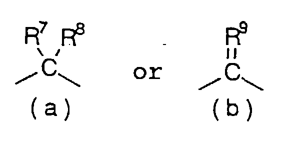
in which each of R⁷ and R⁸ is independently a hydrogen atom, a C₁∼C₄ alkyl group or a C₁∼C₄ alkoxy group, provided that when R⁷ and/or R⁸ are/is C₁∼C₄ alkyl group(s) or C₁∼C₄ alkoxy group(s), one to 9 halogen atoms may be substituted on carbon atoms of the substituents, that when the number of carbon atoms of each substituent is C₂∼C₄, each substituent may have an unsaturated bond, and that when both R⁷ and R⁸ are C₁∼C₄ alkyl groups or C₁∼C₄ alkoxy groups, a combination of these substituents may be a 3- to 7-membered ring in which carbon atoms of the substituents bond to each other, and R⁹ is an oxygen atom, a sulfur atom or a C₁∼C₄ alkoxyimino group, provided that when R⁹ is a C₁∼C₄ alkoxyimino group, one to 9 halogen atoms may be substituted on carbon atoms of the substituent and that when the number of carbon atoms of the substituent is C₂∼C₄, the substituent may have an unsaturated bond.

2. The isoxazole derivative of Claim 1, wherein X is a C₁∼C₄ alkyl group.

3. The isoxazole derivative of Claim 2, wherein X is methyl.

4. The isoxazole derivative of Claim 1, wherein R¹ is cyclopropyl or methyl.

5. The isoxazole derivative of Claim 1, wherein R² is a hydrogen atom.

6. The isoxazole derivative of Claim 1, wherein both R⁵ and R⁶ are hydrogen atoms.

7. The isoxazole derivative of Claim 6, wherein both R³ and R⁴ are hydrogen atoms.

8. The isoxazole derivative of Claim 6, wherein both R³ and R⁴ are methyl.

9. The isoxazole derivative of Claim 1, wherein n is 2.

10. The isoxazole derivative of Claim 3, wherein Y is a hydrogen atom and Z is the group of (a).

11. The isoxazole derivative of Claim 3, wherein Y is a hydrogen atom, z is the group of (b) and R⁹ is an oxygen atom or a sulfur atom.

12. The isoxazole derivative of Claim 1, wherein the isoxazole derivative has the general formula (Ia), 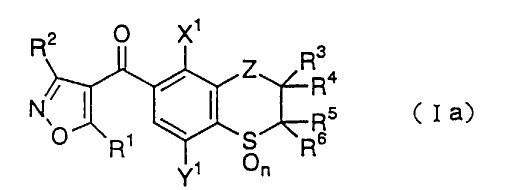 wherein:
R¹ is a C₁-C₆ alkyl group or C₃-C₆ cycloalkyl group,
R² is a hydrogen atom or a C₁∼C₄ alkoxycarbonyl group,
each of R³ ∼ R⁶ is independently a hydrogen atom or a C₁∼C₄ alkyl group,
n is an integer of 0, 1 or 2,
X¹ is a C₁∼C₄ alkyl group,
Y¹ is a C₁∼C₄ alkyl group or a halogen atom, and
Z is a group of 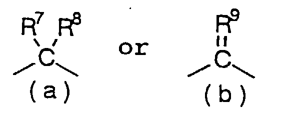
in which each of R⁷ and R⁸ is independently a hydrogen atom, a C₁∼C₄ alkyl group or a C₁∼C₄ alkoxy group, provided that when R⁷ and/or R⁸ are/is C₁∼C₄ alkyl group(s) or C₁∼C₄ alkoxy group(s), one to 9 halogen atoms may be substituted on carbon atoms of the substituents, that when the number of carbon atoms of each substituent is C₂∼C₄, each substituent may have an unsaturated bond, and that when both R⁷ and R⁸ are C₁∼C₄ alkyl groups or C₁∼C₄ alkoxy groups, a combination of these substituents may be a 3- to 7-membered ring in which carbon atoms of the substituents bond to each other, and R⁹ is an oxygen atom, a sulfur atom or a C₁∼C₄ alkoxyimino group, provided that when R⁹ is a C₁∼C₄ alkoxyimino group, one to 9 halogen atoms may be substituted on carbon atoms of the substituent and that when the number of carbon atoms of the substituent is C₂∼C₄, the substituent may have an unsaturated bond.

13. The isoxazole derivative of Claim 1, wherein the isoxazole derivative has the general formula (Ib), 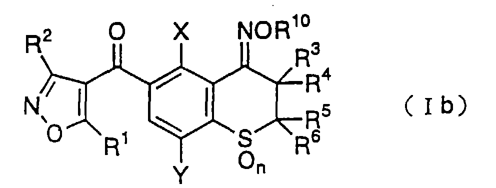 wherein:
R¹ is a C₁-C₆ alkyl group or a C₃-C₆ cycloalkyl group,
R² is a hydrogen atom or a C₁∼C₄ alkoxycarbonyl group,
each of R³ ∼ R⁶ is independently a hydrogen atom or a C₁∼C₄ alkyl group,
n is an integer of 0, 1 or 2,
X is a hydrogen atom or a C₁∼C₄ alkyl group,
Y is a hydrogen atom, a C₁∼C₄ alkyl group or a halogen atom, and
R¹⁰ is a C₁∼C₄ alkyl group provided that one to 9 halogen atoms may be substituted on carbon atoms of the substituent and that when the number of carbon atoms of the substituent is C₂∼C₄, the substituent may have an unsaturated bond.

14. The isoxazole derivative of Claim 12, wherein X¹ is methyl.

15. The isoxazole derivative of Claim 13, wherein X is a C₁∼C₄ alkyl group.

16. The isoxazole derivative of Claim 15, wherein X is methyl.

17. The isoxazole derivative of Claim 12, wherein Y¹ is methyl.

18. The isoxazole derivative of Claim 12, wherein Y¹ is a halogen atom.

19. The isoxazole derivative of Claim 13, wherein Y is a hydrogen atom.

20. The isoxazole derivative of Claim 13, wherein Y is methyl.

21. The isoxazole derivative of Claim 12 or 13, wherein R¹ is cyclopropyl.

22. The isoxazole derivative of Claim 12 or 13, wherein R² is a hydrogen atom.

23. The isoxazole derivative of Claim 12 or 13, wherein both R⁵ and R⁶ are hydrogen atoms.

24. The isoxazole derivative of Claim 23, wherein both R³ and R⁴ are hydrogen atoms.

25. The isoxazole derivative of Claim 12, wherein both R³ and R⁴ are methyl.

26. The isoxazole derivative of Claim 12 or 13, wherein n is 2.

27. The isoxazole derivative of Claim 12, wherein Z is the group of (a), and both R⁷ and R⁸ are C₁∼C₄ alkyl groups.

28. The isoxazole derivative of Claim 12, wherein Z is the group of (a), R⁷ is a C₁∼C₄ alkoxy group and R⁸ is a hydrogen atom.

29. The isoxazole derivative of Claim 12, wherein Z is the group of (a) and both R⁷ and R⁸ are C₁∼C₄ alkoxy groups and form a cyclic acetal group while bonding to each other.

30. The isoxazole derivative of Claim 12, wherein Z is the group of (b) and R⁹ is an oxygen atom.

31. A herbicide containing, as an active ingredient, the isoxazole derivative recited in any one of Claims 1 ∼ 30.
